# EUROPEAN PATENT SPECIFICATION

(11) **EP 4 392 105 B1**
(45) Date of publication and mention of the grant of the patent: **08.04.2026**
(21) Application number: 21790606.4
(22) Date of filing: 27.08.2021
(51) Int. Cl.: A61M 5/315, A61M 5/31

(54) **INJECTOR DEVICE COMPONENT SURFACE MODIFICATION**
OBERFLÄCHENMODIFIKATION EINER INJEKTORVORRICHTUNGSKOMPONENTE
MODIFICATION DE SURFACE DE COMPOSANT DE DISPOSITIF D'INJECTEUR

(43) Date of publication of application: 03.07.2024
(73) Proprietor: W. L. Gore & Associates, Inc., Newark, DE 19711 (US)
(72) Inventor: CULLY, Edward H., Newark, Minnesota 19711 (US); HARDIE, William G., Newark, Delaware 19711 (US)
(74) Representative: HGF
(86) International application number: PCT/US2021/047959
(87) International publication number: WO 2023/027729

(56) References cited:
- WO-A1-2004/044464
- US-A1- 2017 203 046
- US-A1- 2021 146 054

## Description

### FIELD

Various inventive concepts addressed in this description relate to injector devices, such as syringes, auto-injectors, and pens, that include a barrel and a stopper slidably received in the barrel, as well as associated methods of making and using such devices.

### BACKGROUND

Injector devices (e.g., syringes, auto-injectors and pens) typically include a barrel, a stopper positioned within the barrel, and a plunger rod or actuation mechanism to displace the stopper. The stopper is typically air and liquid impermeable while also possessing low-friction slidability. Air impermeability and liquid impermeability are important for eliminating liquid leakage within the barrel and the introduction of air between an outer face of the stopper and an inner wall of the barrel when charging or discharging the liquid inside the injector device. Low-friction slidability is important for facilitating the charging and discharging of the liquid inside the injector device. In addition to these requirements, a medical syringe, auto-injector, or pen should not adversely affect any pharmaceutical composition such as biopharmaceuticals that come in contact with the syringe (e.g., a pre-filled syringe, auto-injector, or pen comprising a pharmaceutical composition).

Some examples of injector device components can be found in U.S. Publication 2021/0030970 by Applicant W. L. Gore & Associates Inc. entitled, "Medical Injector devices Having Low Lubricant Hydrophobic Syringe Barrels," which describes medical injector devices. The medical injector device includes a barrel and a stopper that can provide air and liquid impermeability while also possessing on or more of a low break loose force, a low average glide force, and a low glide force variation.

Additional examples of injector device components can be found in U.S. Patent 10,751,473 by Applicant Sumitomo Rubber Industries, Ltd. entitled, "Gasket, and Medical Syringe," which describes gaskets used for a medical syringe that include a body made of an elastic material and an inert resin film provided on a surface of the body. The gasket has a cylindrical shape, and includes annular ribs provided on an outer circumferential surface thereof, each having a sliding contact portion to be kept in sliding contact with an inner peripheral surface of a syringe barrel. The annular ribs are axially arranged from a distal end to a rear end of the gasket. The sliding contact portion of a distal annular rib has a width that is 1 to 25% of axial length of the cylindrical gasket. WO2004/044464 A1 describes a gasket capable of providing an excellent slidability and assuring sufficient sealability and airtighteness and installed so as to be slidably moved in an outer tube. The gasket comprises a gasket body and having ringshaped projected parts formed on the outer peripheral part thereof, a resin film installed so as to cover the tip face and the projected parts of the gasket body and formed of a material lower in the coefficient of friction than the structure material (elastic material) of the gasket body, and a filler material adhered to the outer surface of the resin film. US2017203046 A1 describes a syringe for storing and delivering a fluid that includes a stopper and a plunger rod assembly. The plunger rod assembly includes a plunger rod and a threaded member at a plunger-engaging end. US2021/146054 A1 describes a medical device that includes a lubricant-free (e.g., silicone) or a siliconized barrel having an interior surface defining a fluid chamber, an elastomeric stopper, and a plunger rod.

### SUMMARY

Forming a durable seal can be difficult for any stopper that includes a barrier, or barrier layer, and does not use silicone or other, additional lubricious material (e.g., liquid lubricant) to fill in defects in the barrier. These defects can be caused by wrinkles that form in the barrier due to compression of the stopper during insertion, from scratches in the surface of the sealing area that occur during manufacturing or insertion of the stopper, or other defects resulting from the component manufacturing and assembly processes. Often times, defects are not created, or do not become apparent, until the stopper is inserted into the barrel. Therefore, it may not be possible to prevent, eliminate, or treat various defects prior to the stopper insertion process into the barrel. Various inventive concepts addressed in this description relate to treating such defects or improving sealing performance during or after a stopper has been through an associated insertion process into a barrel.

According to some examples, a method for manufacturing an injector device includes positioning a stopper of the injector device in a tubular member such that an outer side of the stopper is engaged with an inner surface of the tubular member and modifying the outer side of a stopper by causing relative motion between the stopper and the tubular member, the relative motion including one or both of rotational motion and vibrational motion. The tubular member may be a barrel of the injector device, a vent tube configured to be inserted into a barrel of the injector device, the vent tube being configured for delivery of the stopper into a barrel of the injector device, or another tubular member. The outer side of the stopper optionally defines a rib and the relative motion between the stopper and the tubular member results in localized heating at the rib. In some examples, the relative motion causes a reduction in roughness of the outer side of the stopper. The outer side of the stopper may include wrinkles and the relative motion may cause a reduction in the wrinkles. The outer side of the stopper may define a roughness in a longitudinal direction and a circumferential direction, and the relative motion may cause a reduction in roughness in the longitudinal direction and/or the circumferential direction. The relative motion may cause material from the outer side of the stopper to be transferred to the inner surface of the tubular member. The outer side of the stopper optionally includes polymeric material and the relative motion induces polymeric movement of the polymeric material. The stopper may include a barrier formed of a first material and a body formed of a second material, the barrier being coupled to the body. And, the outer side of the stopper may include a fluoropolymer material. In some examples, the relative motion between the stopper and the tubular member includes a longitudinal component.

According to some examples, a method for manufacturing an injector device includes positioning a stopper of the injector device in a vent tube, inserting the vent tube into a barrel of the injector device, delivering the stopper from the vent tube into the barrel of the injector device such that an outer side of the stopper is engaged with an inner surface of the barrel to define a seal interface between the outer side of the stopper and the inner surface of the barrel, and inducing relative motion between the stopper and the barrel to enhance the seal interface between the outer side of the stopper and the inner surface of the barrel. The relative motion between the stopper and the tubular member may include a rotational component. Optionally, the relative motion between the stopper and the tubular member includes a longitudinal component. The outer side of the stopper may include a polymeric material and enhancing the seal interface between the outer side of the stopper and the inner surface of the barrel includes inducing polymeric movement of the polymeric material at the seal interface. And, as another optional step, after the stopper is delivered from the vent tube into the barrel of the injector device, the outer side of the stopper includes wrinkling at the seal interface, and further wherein enhancing the seal interface between the outer side of the stopper and the inner surface of the barrel includes reducing wrinkling at the seal interface. The stopper may include a body and a barrier coupled to the body, the barrier being formed of a fluoropolymer material, and enhancing the seal interface between the outer side of the stopper and the inner surface of the barrel may include causing localized heating at the seal interface. And, enhancing the seal interface between the outer side of the stopper and the inner surface of the barrel may include transferring material from the outer side of the stopper to the inner side of the barrel. In various examples, the outer side of the stopper defines a rib and the seal interface includes the rib of the stopper.

According to still other examples, an injector device includes a stopper having an outer side and including a body and a barrier formed of a material different than that of the body, the barrier being coupled to the body, the barrier defining at least a portion of the outer side of the stopper and a barrel having an inner surface engaged with outer side of the stopper to define a seal interface, the inner surface of the barrel including a deposited material at the seal interface corresponding to the material of the barrier such that the seal interface is defined by the deposited material and the material of the barrier, the deposited material having a directional orientation. The barrier includes a fluoropolymer material. The deposited material at the seal interface is material that has been transferred from the outer side of the stopper to the inner surface of the barrel by relative motion between the stopper and the barrel. The directional orientation optionally includes a circumferential component. The directional orientation of the deposited material may be defined by rows of PTFE chains aligned in a common direction. The outer side of the stopper optionally includes at least one of a micro rib and a macro rib at the seal interface and the barrier optionally includes a fluoropolymer material. In some examples, the deposited material fills one or more defects in the inner surface of the barrel. The deposited material is optionally only located at the seal interface. The seal interface may correspond to one or more circumferential bands where the outer side of the stopper engages the inner surface of the barrel. And, the deposited material optionally defines one or more circumferential bands on the inside of the barrel.

The foregoing Examples are just that, and should not be read to limit or otherwise narrow the scope of any of the inventive concepts otherwise provided by the instant disclosure. While multiple examples are disclosed, still other embodiments will become apparent to those skilled in the art from the following detailed description, which shows and describes illustrative examples. Accordingly, the drawings and detailed description are to be regarded as illustrative in nature rather than restrictive in nature.

### BRIEF DESCRIPTION OF THE DRAWINGS

The accompanying drawings are included to provide a further understanding of the disclosure and are incorporated in and constitute a part of this specification, illustrate embodiments, and together with the description serve to explain the principles of the disclosure.
FIG. 1 shows an injector device configured as a syringe, according to some embodiments.
FIG. 2 shows an injector device configured as an auto-injector, according to some embodiments.
FIG. 3 shows a stopper of the injector device of FIGS. 1 or 2, according to some embodiments.
FIG. 4 shows a stopper of the injector device of FIGS. 1 or 2, according to some embodiments.
FIG. 5 shows a portion of the stopper of FIGS. 3 or 4, according to some embodiments.
FIGS. 6 and 7 represent various micro features in the area A of FIG. 5, according to some embodiments.
FIGS. 8 and 9 represent tooling and methods by which the tooling can be used for stopper assembly and coupling, according to some embodiments.
FIGS. 10 to 14 are illustrative of some methods of assembling the injector device of FIGS. 1 or 2, according to some embodiments.
FIGS. 15A and 15B represent systems and methods for modifying a stopper, according to some embodiments.
FIGS. 16A to 19B represent modification of the stopper to remove defects, according to some embodiments.

### DETAILED DESCRIPTION

### Definitions and Terminology

This disclosure is not meant to be read in a restrictive manner. For example, the terminology used in the application should be read broadly in the context of the meaning those in the field would attribute such terminology.

The use of headings is provided for ease of review of the description only, and are not meant to segregate or otherwise designate that concepts under one heading are inapplicable or otherwise unrelated to concepts under another heading. In fact, the opposite is intended and the description is meant to be read and interpreted as a whole, with various features and aspects of certain embodiments being applicable across and applicable to the various other embodiments described herein.

With respect to terminology of inexactitude, the terms "about" and "approximately" may be used, interchangeably, to refer to a measurement that includes the stated measurement and that also includes any measurements that are reasonably close to the stated measurement. Measurements that are reasonably close to the stated measurement deviate from the stated measurement by a reasonably small amount as understood and readily ascertained by individuals having ordinary skill in the relevant arts. Such deviations may be attributable to measurement error, differences in measurement and/or manufacturing equipment calibration, human error in reading and/or setting measurements, minor adjustments made to optimize performance and/or structural parameters in view of differences in measurements associated with other components, particular implementation scenarios, imprecise adjustment and/or manipulation of objects by a person or machine, and/or the like, for example. In the event it is determined that individuals having ordinary skill in the relevant arts would not readily ascertain values for such reasonably small differences, the terms "about" and "approximately" can be understood to mean plus or minus 10% of the stated value.

As used herein, the terminology "activatable by an energy source" and its analogs refer to a change of state of a material, such as a change in physical and/or chemical state. One example of activation by an energy source includes a marked (i.e., clearly evident) change from a solid form (or more solid form) to a liquid form (or more liquid form). Another example of activation by an energy source includes exhibiting a marked (i.e., clearly evident) change in cross-linking or molecular weight (e.g., via cross-linking or chain scission) through exposure to an energy source. For reference, as used herein, "energy source" refers to sources of any of a variety of types of energy, including thermal, laser, radiofrequency (RF), microwave, ultraviolet, radiant, ultrasound, and others.

As used herein, the terms "barrier," "barrier construct," or the like refer to material that blocks or hinders interaction between one component (e.g., a stopper body) and another (e.g., a barrel and/or the contents of a barrel).

As used herein, the terms "elastic" and "elastomeric" refer to a material property understood with reference to stoppers employed in injector devices (e.g., in FDA-approved applications) and relates to the tendency of a material to spontaneously revert back, or recover, toward its pre-deformation shape after being dimensionally deformed (e.g., contracted, dilated, distorted, or the like).

As used herein, the term "injector device" is meant to be inclusive of any of a variety devices that include a stopper received in a barrel and an actuation mechanism configured to displace the stopper within the barrel to eject, or deliver contents held in the barrel from within the barrel. Examples of injector devices include syringes, auto-injectors, and pens.

As used herein, the term ""macro feature" (e.g., as in "macro rib" or "macro groove") is meant to denote a stopper rib or groove feature, the contours of which are visible with the naked eye, or a stopper feature that exhibits a height that is two or more times the thickness of the barrier of the stopper.

As used herein the term "micro feature" (e.g., such as a micro rib, micro groove, or micro void) is meant to denote a stopper feature (whether a surface feature or subsurface feature), the contours of which are not visible with the naked eye (though the general existence of the feature may itself be appreciable). For example, a micro feature would include a micro rib or micro groove feature of a stopper that is located on or in a macro rib or macro groove.

As used herein, the term "multi-layer barrier" refers to a barrier construct that has a plurality of layers of material, at least portions of which are arranged in a superimposed fashion one over the other (a parallel arrangement), or in some cases, one adjacent the other (a series arrangement). A multi-layer construct may have thicknesses or layers of material with relatively sharp, distinct boundaries, or may have blended or more gradual transition boundaries therebetween.

As used herein, the term "multi-zone barrier" refers to a barrier construct that has a plurality of zones, or sections having different material properties. A multi-zone construct may have zones, or sections separated by relatively sharp, distinct boundaries, or may have blended or gradual boundaries. Some examples of multi-zone barriers include distinct layers arranged in parallel or in series, such that a multi-layer barrier also defines a multi-zone barrier. Other examples may include a single layer that is modified to define multiple zones.

As used herein, the term "oscillate" and the like (e.g., "oscillation") is meant to denote motion that alternates in direction at a frequency that may be constant or varying.

As used herein, the term "proximal" means closer to the operator end of a device (e.g., plunger end) while the term distal means further away from the operator than proximal (e.g., piercing element end).

As used herein, the term "rotate" and the like (e.g., "rotation") is meant to denote circumferentially-oriented motion.

As used herein, the term "sealing surface" is meant to denote a feature that maintains a liquid-tight seal (e.g., in storage and/or in use).

As used herein, the terms "silicone" and "silicone oil" may be used interchangeably herein.

As used herein, the term "substantially free" is meant to denote an unquantifiable or trace amount of the identified substance (e.g., silicone, silicone oil, or other lubricant), or that there is not any amount intentionally added to the system (e.g., no silicone oil intentionally added to an injector device, such as the barrel or stopper).

As used herein, the term "vibrate" (e.g., "vibration") is meant to denote motion that alternates having an acceleration that alternates in direction at a frequency that may be constant or varying.

### Description of Various Embodiments

Persons skilled in the art will readily appreciate that various aspects of the present disclosure can be realized by any number of methods and apparatuses configured to perform the intended functions. It should also be noted that the accompanying drawing figures referred to herein are not necessarily drawn to scale, but may be exaggerated to illustrate various aspects of the present disclosure, and in that regard, the drawing figures should not be construed as limiting.

The present disclosure is directed to injector devices (e.g., syringes, auto-injectors, and pens) that include a stopper at least partially covered with a fluoropolymer or non-fluoropolymer film or fluoropolymer or non-fluoropolymer laminate, a barrel, and a plunger rod or actuation mechanism to displace the stopper within the barrel.

Various aspects of this description relate to mechanical modification of a stopper while it resides within a tubular member, such as a barrel of an injector device or a vent tube operable for inserting a stopper into a barrel of an injector device. The stopper may have a barrier, and the stopper may or may not have at least one micro feature (e.g. formed by the barrier). Such barriers may include multiple layers, or be a multi-layer barrier. Certain defects, such as wrinkles, scratches, and debris may be present in the stopper in its assembled form and/or may be generated or otherwise present during assembly of the stopper into a barrel of an injector device. Mechanical modification of the stopper may be implemented during the manufacturing process (e.g., prior to, or after the stopper is inserted into the barrel) to reduce the effect of such defects and imperfections. Mechanical modification may additionally or alternatively transfer material from stopper to barrel to help reduce the impact of imperfections in the barrel surface and/or generate a sliding interface including the same materials. For example, a PTFE-to-PTFE (or an ePTFE-to-eTPFE interface) formed by transferred material on the barrel and the barrier material on the stopper, may have enhanced sealing and/or sliding performance. It is contemplated that such an interface may have a lower likelihood of leaking based upon the surface energies between the two similar materials.

These various features may, in turn, may help achieve better and/or more repeatable results in sealing and/or sliding performance. Various additional or alternative advantages may be realized leveraging such features, including more efficient and/or higher yield manufacturing, reduced contamination and/or particulate generation, enhanced sealing, or others.

### Injector Device Concepts

In use, the injector devices may be employed for storing (e.g., short term or long term) and delivering a fluid, which is typically a therapeutic or other substance delivered to a patient for medical use. In some embodiments, such injector devices may be pre-filled with a therapeutic (e.g., as a pre-filled syringe) in advance of the planned use of the injector device to deliver the therapeutic to a patient. The injector devices may contain a therapeutic that treats diseases, such as, but not limited to, ocular disease (e.g., macular degeneration and glaucoma) or diabetes. Non-limiting examples of potential therapeutics are subsequently described. Advantageously, in various embodiments, the stoppers and barrels do not contain silicone, or silicone oil. For example, the barrels and stoppers in the injector devices described herein may be free or substantially free of silicone and silicone oil (or other liquid lubricant), according to various embodiments. In some instances, the stoppers and barrels do not contain any substantial amount, or are substantially free of any other liquid lubricant (excluding, of course, therapeutic substances in the injector device that are in liquid form, and thus lubricating themselves to at least some extent).

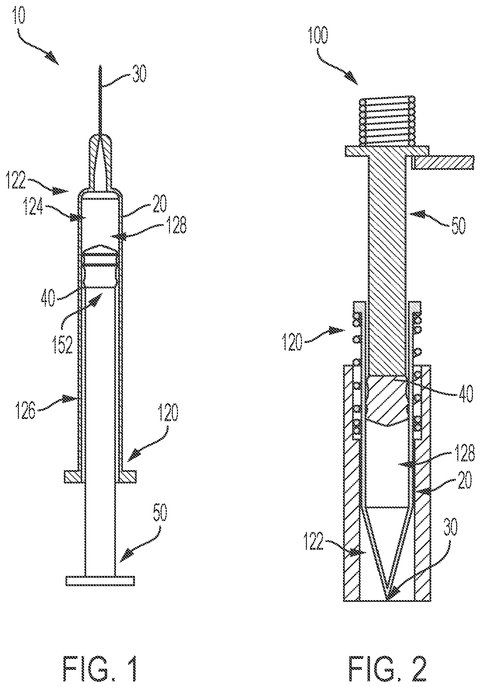

FIG. 1 depicts an injector device 10 in the form of a syringe, according to some embodiments. As shown, the injector device 10 includes a barrel 20, a piercing element 30, and a stopper 40 received in the barrel 20 and operatively coupled to an actuation mechanism 50 (e.g., a plunger rod as shown).

As shown, the barrel 20 has a wall 118 and extends between a proximal end 120 and a distal end 122. The barrel 20 has an inner surface 124 and an outer surface 126 that are each defined by the wall 118 of the barrel 20, the inner surface bounding a receiving chamber 128 defined by the barrel 20. As shown, the proximal end 120 of the barrel 20 may also include a flange that may be used as a finger stopper or handle to assist a user in pressing and pulling the actuation mechanism 50.

The piercing element 30 may include a sharply pointed needle cannulae, or a blunt-ended cannula, such as those employed with "needleless" systems. For ease of illustration, the piercing element 30 is depicted as a sharply pointed, elongate needle cannula with a sharply pointed distal end. As shown, the piercing element 30 is coupled with the distal end 122 of the barrel 20.

The stopper 40 is configured to be slidably received in the barrel 20, and to seal with the inner surface 124 of the barrel 20. More specifically, the stopper 40 is configured to be actuated within the barrel 20 by the actuation mechanism 50 to pressurize and expel contents of the receiving chamber 128 from the barrel 20 through the piercing element 30.

The actuation mechanism 50 has a distal end 152 and a proximal end 154, where the distal end 152 is operatively coupled to the stopper 40, for example being fastened, integrally formed with, or otherwise associated with the stopper 40 in such a manner that the actuation mechanism 50 is configured to displace the stopper 40 within the barrel 20 in a longitudinal (or other) direction.

FIG. 2 depicts an injector device 100 in the form of an auto-injector, according to some embodiments, in which the barrel 20, the stopper 40 and the actuation mechanism 50 (also described as an injection member in association with the injector device 100) may be similarly configured and employed. The actuation mechanism 50 of the injector device 100 may be employ, or exhibit a variable actuation force that is applied to the stopper 40. For example, the actuation mechanism 50 may include one or more biasing members (e.g., springs) and other features for achieving such functionality. Various other components of the injector device 100 are substantially similarly to those of the injector device 10, as would be understood by those in the relevant field of practice. For purposes of this description, the various features of the stopper 40 described herein are applicable whether utilized in the configuration of injector device 10 or that of the injector device 100. In broader terms, the concepts described herein with respect to barrel 20 and stopper 40 may be implemented in any of a variety of injector device configurations.

The injector devices 10, 100 may include a material 60 in the receiving chamber 128 of barrel 20. In some examples, the material 60 is deposited or otherwise positioned in the chamber at a manufacturing site, or a site that is remote from the treatment site or site at which the injector device 10, 100 is to be employed by an end user (e.g., at a clinical site). In such cases, the injector device 10, 100 may be referred to as being "pre-filled" (e.g., in the example of the injector device 10, a prefilled syringe). The material 60 may be a predetermined amount (e.g., one or more doses) of a pharmaceutical composition. Some examples of suitable pharmaceutical compositions are subsequently described. However, it should be understood that the material 60 could be any type of liquid or material capable of being expelled from a syringe, or the material 60 may be all together absent from the receiving chamber, such as in an unfilled syringe. In such examples, the injector devices 10, 100 may be filled at or near a treatment site (e.g., also described as "charging" the injector device).

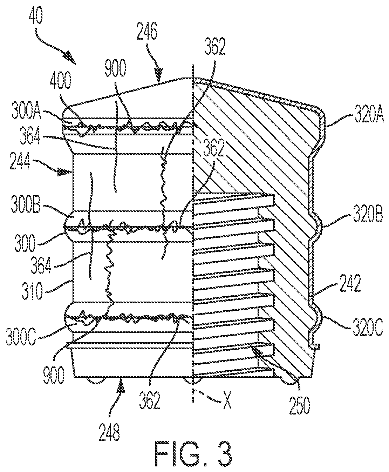

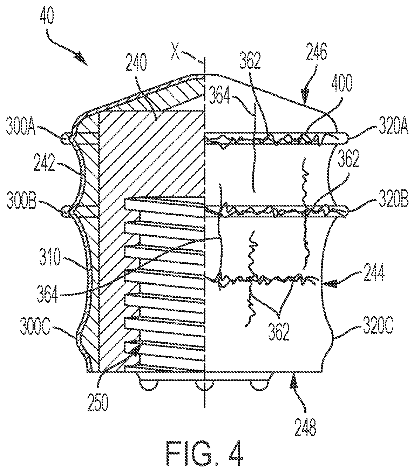

FIGS. 3 and 4 are plan, or front views of example configurations of the stopper 40, with a right half of the stopper 40 illustrated in section in the configuration of FIG. 3 and a left half of the stopper 40 illustrated in section in the configuration of FIG. 4.

As shown in each of the configurations of FIG. 3 and FIG. 4, the stopper 40 includes a body 240 made of an elastic material, and a barrier 242, such as a barrier film, provided on the body 240. The stopper 40 has an outer side 244, a longitudinal axis X, and a height along the longitudinal axis X. The stopper 40 extends between a leading face 246 and a trailing face 248. As shown, the barrier 242 may extend along a portion of (including an entirety of) the outer side 244 and/or the leading face 246. If desired, the barrier 242 may also extend along a portion of (including an entirety of) the trailing face 248.

In some embodiments, the body 240 provides a desired degree of resilient compliance to the stopper 40. For example, the body 240 may be compressed upon insertion of the stopper 40 into the barrel 20 so that the stopper 40 positively engages with the barrel 20. Suitable materials for the body 240 are described further below.

In various examples, the barrier 242 provided on the body 240 is configured to inhibit migration of substances from (or to) the body 240 through the barrier 242, reduce sliding and/or static friction between the stopper 40 and the barrel 20, and/or to enhance sealing between the stopper 40 and the barrel 20. Such features are referred to in the exemplary sense, and are not meant to be an exclusive list. The barrier 242 may be a single layer, or multiple layers. The barrier 242 may be constructed with multiple layers that have unique properties from one another and/or the barrier may include multiple layers with similar properties that are fused or otherwise coupled to form a more homogenous construct with more homogenous properties from layer-to-layer. The barrier 242 may also include composite materials (e.g., a matrix film material and a filler) serving as one or more layers of the barrier 242. Suitable materials for the barrier 242 are described further below.

As shown in each of the configurations of FIGS. 3 and 4, the stopper 40 has a short, cylindrical shape, with the leading face 246 being defined by a conical end of the stopper 40. As shown, the conical end can project away from the longitudinal axis X to define an obtuse angle. In examples where the actuation mechanism 50 is coupled to the stopper 40 using a threaded fastening arrangement, the stopper 40 may include an axial recess 250 in the trailing face 248 with female threading.

As shown, the outer side 244 of stopper 40 may define one or more ribs 300, also described as macro ribs, such as one or more circumferentially extending annular ribs 300 and/or one or more grooves 310, also described as macro grooves 310, such as one or more circumferentially extending annular grooves 310. In operation, one or more of the ribs 300 are configured to engage inner surface 124 (FIGS. 1 and 2) of the barrel 20 in sliding contact. The stopper 40 may be configured to achieve container closure integrity with high levels of gas (e.g., air) and liquid impermeability while also maintaining one or more of: acceptably low break loose force, low average glide force, and low glide force variation.

The ribs 300 can be structured in any number of configurations. For example, only the distalmost or leading rib may have a sealing surface. It is to be appreciated that the quality of a seal thus formed may be assessed by any number of methods familiar to one skilled in the art (e.g. helium leak testing). In some embodiments, multiple ribs 300 may have a sealing surface. In one or more embodiment, all of the ribs 300 having a sealing surface may have a same predefined outer diameter (e.g., measured from an apex of the respective rib with the stopper 40 in a non-compressed state). In other embodiments, each rib 300 having a sealing surface may have its own predefined outer diameter. For example, a distal or leading rib may have a predefined outer diameter and a proximal or trailing rib may have a predefined outer diameter that is between about 75% and about 99.9% of the predefined outer diameter of the distal or leading rib. Other types of rib arrangements are contemplated, such as, for example having three ribs with sealing surfaces.

Although three ribs 300 are shown in FIGS. 3 and 4, any number of ribs (e.g., one, two, four, ten, and so forth) are contemplated. As shown, the ribs 300 include a leading rib 300A having a sealing surface 320A (also described as a sliding contact portion 320A) configured to be in sliding contact with the inner surface 124 of the barrel 20. As shown in FIG. 3, one or more of the ribs 300 optionally has a flattened profile (e.g., the leading rib 300A) in which the sealing surface (e.g.., the sealing surface 320A) may be somewhat flattened, and have a width that is 1 to 25% of the length of the outer side 244 of the stopper 40. As shown in FIG. 4, one or more of the ribs 300 (e.g., the leading rib 300A) optionally has an outwardly convex shape, where the sealing surface (e.g., the sealing surface 320A) has a relative narrower profile. As shown in FIGS. 3 and 4, the ribs 300 also include an intermediate rib 300B and a trailing rib 300C. As shown, the intermediate rib 300B and the trailing rib 300C optionally have an outwardly convex shape as seen in section. Each of the intermediate rib 300B and trailing rib 300C optionally have sealing surfaces 320B, 320C, respectively, that are configured to be in sliding contact with the inner surface 124 of the barrel 20. Where one or more of the ribs 300 have an outwardly convex shape, the corresponding sealing surfaces may have relatively small widths as measured along the longitudinal axis X of the stopper 40. Depending upon configuration, each of the sealing surfaces 320B, 320C (also described as sliding contact portions 320B, 320C) may have widths that are greater than 0% and up to 15% of the length of the outer side 244 of the stopper 40.

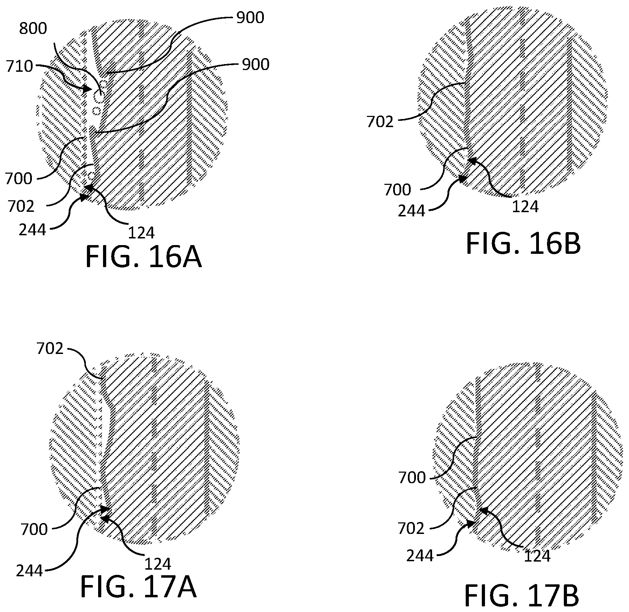

As shown in FIGS. 3 and 4, the outer side 244 of the stopper 40 may include one or more defects 900, such as wrinkles 362 and scratches 364 (examples of defects 900 in the form of debris can be found and described in association with FIG. 16A). The various defects 900, such as the wrinkles 362 and/or scratches 364 may be oriented longitudinally, circumferentially, or both (e.g., helically). The defects 900 may be relatively linear, curved, or both. The defects may be located at any location on the stopper 40, but may be particularly prevalent on the ribs 300 and the associated sealing surfaces 320, as well as on or along one or more micro features 400, such as those subsequently described. These defects may be formed at any point in the manufacturing process, including when the stopper 40 is first formed (e.g., when the barrier 242 is attached to the body 240) or during the process of installing the stopper 40 into the barrel 20. For example, the wrinkles 362 may be formed when the stopper is diametrically compressed. And, the scratches 364 may be formed when the stopper 40 is slid against the barrel 20 or another tubular member utilized during the assembly process, for example.

As shown in FIGS. 3 and 4, the outer side 244 of the stopper 40 may include one or more defects 900, such as wrinkles 362 and scratches 364 (examples of defects 900 in the form of debris can be found and described in association with FIG. 16A). The various defects 900, such as the wrinkles 362 and/or scratches 364 may be oriented longitudinally, circumferentially, or both (e.g., helically). The defects 900 may be relatively linear, curved, or both. The defects may be located at any location on the stopper 40, but may be particularly prevalent on the ribs 300 and the associated sealing surfaces 320, as well as on or along one or more micro features 400, such as those subsequently described. These defects may be formed at any point in the manufacturing process, including when the stopper 40 is first formed (e.g., when the barrier 242 is attached to the body 240) or during the process of installing the stopper 40 into the barrel 20. For example, the wrinkles 362 may be formed when the stopper is diametrically compressed. And, the scratches 364 may be formed when the stopper 40 is slid against the barrel 20 or another tubular member utilized during the assembly process, for example.

### Micro Feature Concepts

As designated in FIGS. 3 and 4, the stopper 40 includes one or more micro features 400 located at one or more of the ribs 300, such as at the sliding contact portion 320A of the leading rib 300A. In some examples, the one or more micro features 400 include one or more micro grooves and/or micro ribs. In some examples, the micro feature 400 has a width and a depth, where depth is the amount of projection in the case of a micro rib and the amount of recess in the case of a micro groove. In some embodiments, one or both of the width and the depth are not greater than 200 µm, not greater than 100 µm, not greater than 50 µm, not greater than 10 µm, or not greater than 5 µm for example, though a variety of dimensions are contemplated. Note that each of the foregoing "not greater than" ranges includes a value greater than "zero".

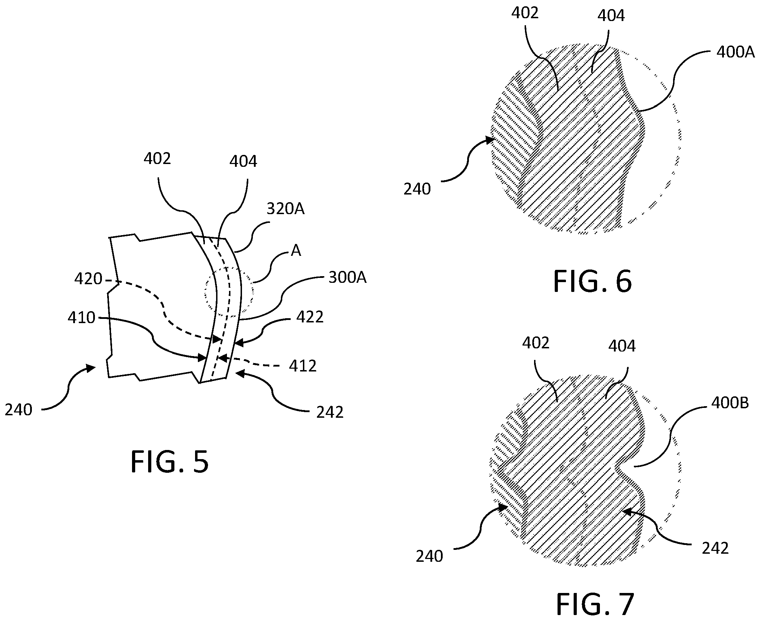

FIG. 5 is representative of an enlarged, sectional view of one or more portions of the stopper 40 along the outer side 244 of the stopper 40 (e.g., at one of the ribs 300, such as the first rib 300A). Although FIG. 5 shows the first rib 300A, the same concepts described in association with the first rib 300A may apply to any of the ribs 300. FIG. 6 and 7 represent various micro features optionally included in the area "A" noted on FIG. 5. The micro features may be formed by the barrier 242 and/or the body 240.

With the foregoing in mind, FIG. 5 shows a section of the body 240 and barrier 242 of the stopper 40, along with the barrel 20 (e.g., at a location where the outer side 244 of the stopper 40 engages with the inner surface 124 of the barrel 20), according to some embodiments. As shown, the barrier 242 optionally includes a plurality of layers, or is a multi-layer barrier including a first layer 402 of a first material and a second layer 404 of a second material. The barrier 242 may have any of a variety of thicknesses, such as between 1 µm and 200 µm.

As shown, the first layer 402 may be positioned under the second layer 404, where present. Although two layers are generally illustrated, it should be understood that any number of layers are contemplated, including a single layer. As shown, the first layer 402 has an inner surface 410 facing toward the body 240 of the stopper 40 and an outer surface 412 facing toward the second layer 404. The second layer 404, in turn, includes an inner surface 420 facing toward the first layer 402 and an outer surface 422 facing away from the body 240. In various examples, the inner surface 410 of the first layer 402 is coupled (e.g., bonded, adhered, fastened, or otherwise coupled) to the body 240. And, in turn, the inner surface 420 of the second layer 404 is coupled (e.g., bonded, adhered, fastened, or otherwise coupled) to the first layer 402. In some embodiments, the first layer 402 can be referred to as an "inner layer" and the second layer 404 can be referred to as an "outer layer" of the barrier 242, although either of the first layer 402 and/or the second layer 404 may be an intermediate, or buried layer positioned between one or more other layer(s) of the barrier 242.

In various examples, one of the plurality of layers (e.g., the first layer 402) may include a first material that is more activatable by an energy source than a second material of another of the plurality of layers (e.g., the second layer 404). In particular, this feature of one layer being more activatable by an energy source than another may be leveraged to preferentially form a variety of micro features 400 in the barrier 242 at a variety of locations.

A variety of materials are contemplated the barrier 242, including those separately described. For example, the barrier 242 (e.g., the first material and/or the second material) may include a fluoropolymer (e.g., polytetrafluoroethylene (PTFE) or expanded PTFE (ePTFE)). In some examples, the first layer 402 is microporous and defines a first porosity and the second layer 404 has a lower porosity than the first layer, and, optionally, the second layer 404 is characterized by a higher melt temperature than the first layer 402. If desired, the second layer 404 may be characterized by a higher dimensional stability than the first layer 402. At least one of the first material of the first layer 402 and the second material of the second layer 404 may include a thermoplastic material. If desired, the first material of the first layer 402 may include a filler configured to increase absorption of light energy and/or radiofrequency energy of the first material. And, the filler may include at least one of fluorinated ethylene propylene (FEP) and ethylene tetrafluoroethylene (ETFE), for example.

FIGS. 6 and 7 show examples of micro features. FIG. 6 shows an example of a potential micro feature 400 in the form of a micro rib 400A and FIG. 7 shows a potential micro feature in the form of a micro groove 400B, or micro void 400B. Although FIGS. 6 and 7 each show a micro feature example, it should be understood that any number of micro features may be present or any combination of micro features for the stopper 40 are contemplated. Example methods of forming such micro features would include directing an energy source at the barrier 242 to form such features, pre-molding or molding such features, or any of a variety of other methods of formation.

Following formation of the various micro features (e.g., micro voids, micro grooves, or micro ribs) at or near the particular microfeature 400 the barrier 242 generally, and the first layer 402 and/or second layer 404 more specifically, may exhibit relatively different physical properties than surrounding portions of the barrier 242, such as one or more of: increased compliance in the case of micro voids or micro grooves; reduced compression resistance in the case of micro voids or micro grooves; increased compression resistance in the case of micro ribs, reduced thickness in the case of micro voids or micro grooves; increased thickness in the case of micro ribs, or reduced tensile strength in the case of micro voids or micro grooves. Such characteristics may be advantageous in reducing an effective sealing surface area of a rib 300 (e.g., to optimize the relationship between increased sealing force and reduced sliding resistance of the macro rib), creating a preferential failure line for the barrier 242 (e.g., to pre-select a more desirable area for the barrier to tear or fail to avoid contamination of the contents of injector device 10 and/or seal failure), to fill one or more voids or defects between the barrel 20 and the stopper 40 or other advantages in performance and reliability.

Although previously referenced, for the avoidance of doubt the various multi-layer barrier configurations, may include more than two layers (e.g., five in total). The first layer 402 and/or the second layer 404 may be as described in association with prior examples may be at any position within the layers. And, there may be greater or fewer layers in various implementations. The first layer 402 may be an innermost layer, or a buried layer, for example. The second layer 404 may be an outermost layer, or a buried layer, for example. And, the first layer and second layers 402, 404 may be in contact, or separated by one or more other layers. And, a single layer may be present consistent with various embodiments.

The various micro features 400 described above may have any of a variety of dimensions. In some examples, one or more of the micro grooves have a depth from 0.25 µm to 50 µm, and optionally from 0.25 µm to 0.5 µm and a width from 0.25 µm to 50 µm, and optionally from 0.25 µm to 0.5 µm and/or one or more of the micro ribs has a height from 0.25 µm to 50 µm, and optionally from 0.25 µm to 0.5 µm and a width from 0.25 µm to 50 µm, and optionally from 0.25 µm to 0.5 µm. As will be subsequently described, the micro grooves and/or micro ribs may have any of a variety of configurations, for example extending in a circumferential direction, a helical direction, or even a longitudinal direction.

### Stopper Assembly and Coupling Mechanisms

Various manners of assembly the stopper, and in particular arranging the barrier 242 and the body 240 together, are contemplated.

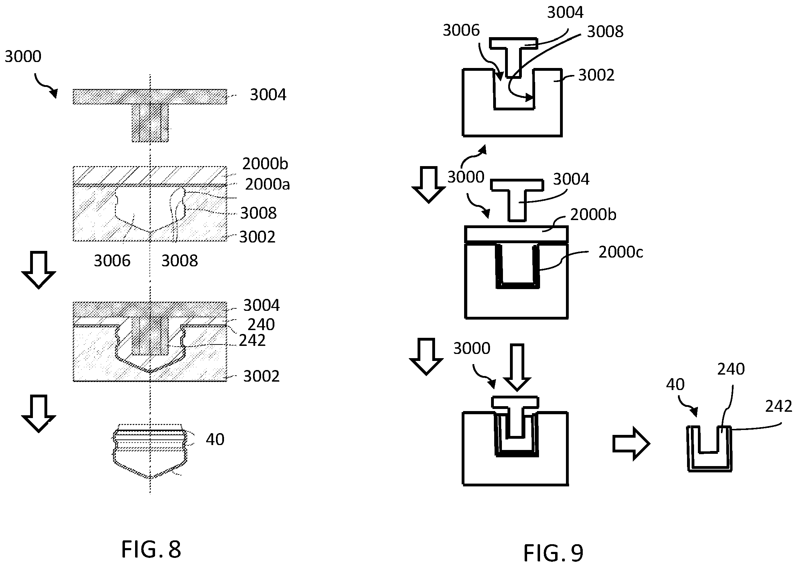

For example, FIG. 8 includes the use of tooling 3000, including a mold 3002 and a forming apparatus such as mandrel 3004. The mold 3002 includes a cavity 3006 defined by an interior wall 3008. The cavity 3006 is shaped and sized to produce the stopper 40 with a desired shape and size. As shown, tooling 3000 is configured to manufacture the stopper 40 from a preform 2000a of barrier material and a preform 2000b of body material, each of the preforms 2000a, 2000b being in sheet, or relatively planar form to start.

The preforms 2000a, 2000b are optionally aligned and then forced (e.g., simultaneously) into the cavity 3006 of the mold 3002 as shown. The body 240 is thereby formed from the preform 2000b with the barrier 242 co-molded or laminated thereon from the preform 2000a to form the stopper 40 as shown. In the illustrated embodiments, the mandrel 304 is actuated to force the preforms 2000a, 2000b into the mold 3002. In some embodiments, the mandrel 3004 can be configured to define a structure in body 240 during formation (e.g., the axial recess 250 in the trailing face 248 with female threading).

Injection molding, compression molding, vacuum press molding, co-molding or other known or otherwise conventional processes and equipment can also be used to manufacture the stopper 40 using the preforms 2000a, 2000b.

As another example, FIG. 9 is illustrative of some embodiments how a preform 2000c of the material of the barrier 242 in a cylindrical form can be combined with a preform 2000b of the material of the body 240 in a sheet form to assemble the stopper 40. As shown in FIG. 9, the process includes use of tooling 3000 including a mold 3002 and a forming apparatus such as mandrel 3004. The mold 3002 includes a cavity 3006 defined by an interior wall 3008. The cavity 3006 is shaped and sized to produce the stopper 40.

Tooling 3000 is configured to manufacture the stopper 40 from the preform 2000c of barrier material and a mass body material defining the preform 2000b. As shown, the preform 2000c of barrier material is positioned in the cavity 3006 of the mold 3002. The preform 2000b of body material is then applied to the interior void area within the preform 2000c of barrier material. As shown, the mandrel 3004 is actuated to force the preform 2000b, which can be in a solid or semi-solid form, into the preform 2000c through the open proximal end portion of the preform 2000c. The mandrel 3004 can be configured to define a structure in the preform 2000b (e.g., the axial recess 250 in the trailing face 248 with female threading).

Though mandrel 3004 is optionally utilized, in other embodiments the body material is deposited into the preform 2000c of barrier material by other approaches such as in a flowable or other fluid form by the application of pressure. Injection molding, compression molding, vacuum press molding, co-molding or other known or otherwise conventional processes and equipment can be used to manufacture the stopper 40 using the preform 2000c.

Various modifications to the foregoing may be applied to enhance or achieving component bonding. In some examples, the barrier 242 may be bonded (or further bonded) to the body 240 during formation of the one or more micro features 400 or by activating the first layer 402 with the energy source. The additional use of adhesives, elastomeric bonding materials, surface treatments and other practices are also contemplated.

### Stopper Insertion Concepts

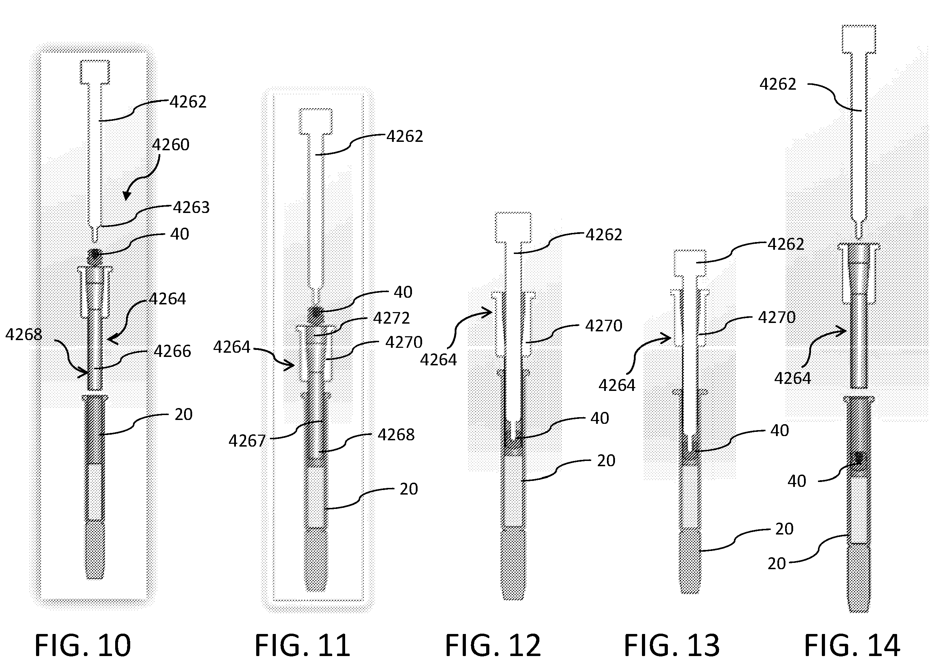

FIGs.10-14 are diagrammatic illustrations of a sequence of steps by which insertion apparatus 4260 can be used to insert the stopper 40 into the barrels 20 which may be pre-filled or subsequently filled with any of a variety of contents, such as any of the therapeutic substances described herein. As shown, the insertion apparatus 4260 includes an insertion pin 4262 and a vent tube 4264. Vent tube 4264 includes an elongated tubular member 4266 having an outer diameter that is less than the inner diameter of the barrel 20, and an inner surface 4267 having an inner diameter that is large enough to accommodate the stopper 40. As perhaps best shown in FIG. 11, the tubular member 4266 of the vent tube 4264 is inserted into the barrel 20 through its proximal end. In some embodiments, a distal end portion 4268 of the vent tube 4264 is located at a position corresponding to the desired position of the stopper 40 in the barrel 20 in the assembled injector device 10, 100. For example, as shown in FIGS. 11 and 12, the distal end portion 4268 of the vent tube 4264 is located adjacent to the surface of syringe contents, such as the therapeutic substance, when the tubular member 4266 is positioned in the barrel 20.

Insertion pin 4262 has an outer diameter that is less than an inner diameter of the vent tube 4264, and a distal end portion 4263. In embodiments, the inner diameter of the vent tube 4264 is less than the outer diameter of the stopper 40. A proximal end portion 4270 of the vent tube 4264 has a tapered interior guide surface 4272. As perhaps best shown by FIGs. 11 and 12, while the vent tube 4264 is positioned in the barrel 20, the insertion pin 4262 is actuated or moved to engage its distal end portion 4263 with the stopper 40 and to force or otherwise drive or move the stopper 40 into the proximal end portion 4270 of the vent tube 4264, and through the tubular member 4266 to the distal end portion 4268 of the vent tube 4264. By this action of the insertion pin 4262, the stopper 40 is diametrically compressed (e.g., as the stopper 40 is moved through the tapered guide surface 4272), and positioned at a position along the length of the barrel 20 that is the desired position of the stopper in the barrel of the assembled injector device 10. 100 (e.g., adjacent a therapeutic substance in the barrel 20).

As perhaps best shown by FIG. 13, while the relative positions of the insertion pin 4262 and the barrel 20 remain fixed, the vent tube 4264 is withdrawn from the proximal end of the barrel 20. The insertion pin 4262 retains the stopper 40 at the desired position in the barrel 20 during this removal of the vent tube 4264, causing the stopper 40 to be urged out of the distal end portion 4268 of the vent tube 4264. After exiting the vent tube 4264, the stopper 40 expands diametrically into engagement with the barrel 20 (e.g., the outer side 244 of the stopper 40 engages the inner surface 124 of the barrel 20 at one or more of the seal interfaces). The stopper 40 is thereby positioned at its desired location in the barrel 20. The insertion pin 4262 and vent tube 4264 can then be withdrawn from the barrel 20 as shown, for example by FIG. 14.

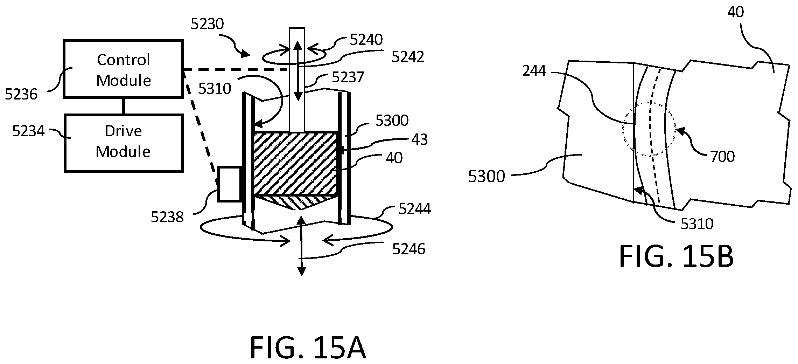

The stopper insertion process described above in connection with FIGS. 10-14, may produce wrinkles or surface defects, as subsequently described. In particular, irregularly shaped and elongated bulges may result adjacent to grooves 310 and/or ribs 300. These irregularly shaped and elongated structures, which may be referred to herein as wrinkles or surface defects 900 (FIGS. 3, 16A, 18A, and 19A), may have substantial components in directions generally parallel, perpendicular, or at any angle relative to the longitudinal axis X at seal interface 702 (FIG. 15B) following the insertion of the stopper 40 into the barrel 20. The wrinkles or surface defects 900 (FIG. 3) may detract from or negatively impact sealing characteristics of the seal interface. For example, they may function as channels that allow the ingress or egress of undesired gasses and/or liquids past the stopper 40.

As previously referenced, modification of the stopper 40 within the barrel 20 may facilitate reduction of such wrinkles or surface defects 900 (FIG. 3) and/or generally enhance sealing between the stopper 40 and the barrel 20.

### Modification System and Method Concepts

FIG. 15A is a diagrammatic illustration of modification system 5230 that can be used to manufacture injector devices 10 including stoppers 14 in accordance with embodiments. Modification system 5230 can be used to perform processing of the stopper 40 and/or barrel 20 (e.g., before the stopper 40 is inserted into the barrel 20, while the stopper 40 is being inserted into the barrel 20, and/or after the stopper 40 has been inserted into the barrel 20). Various examples relate to a method for manufacturing injector device 10, 100 including positioning the stopper 40 of the injector device 10, 100 in a tubular member 5300 (e.g., barrel 20 or vent tube 4264) such that the outer side 244 of the stopper 40 is engaged with an inner surface 5310 (e.g., inner surface 124 of barrel 20 or inner surface 4267 of vent tube 4264). The stopper 40 may engage with the inner surface 124 of the barrel 20 in any of a number of ways, including through use of the vent tube 4264. And, the stopper 40 may be engaged with the inner surface 4267 of the vent tube 4264 in any of a number of ways, including those described in association with FIGS. 10 to 14.

In various examples, modifying the outer side 244 of a stopper 40 includes causing relative motion between the stopper 40 and the inner surface 5310 of the tubular member 5300, the relative motion including one or both of rotational motion and linear motion. Such rotational motion may be through any number of degrees of rotation, such as 5 or more degrees, 10 or more degrees, 20 or more degrees, 40 or more degrees, 100 or more degrees, 180 or more degrees, 360 or more degrees, more than 360 degrees, 720 or more degrees, or any other value or range between the aforementioned values. The relative motion may be oscillatory (e.g., vibrational) in nature or continuous or discontinuous. The oscillatory relative motion may occur at any desired frequency, such as 0.5 Hz or more, 1 Hz or more, 5 Hz or more, 10 Hz or more, 20 Hz or more 50 Hz or more, 100 Hz or more, or any other value or range between the aforementioned values. The modification system 5230 may be configured to impart such relative motion. The relative motion may cause localized heating, or in more general terms generate energy, at the interface(s) between the stopper and the tubular member (e.g., syringe barrel 20 or vent tube).

In various embodiments, the modification system 5230 providing relative movement between the barrel 20 and stopper 40 enhances characteristics of the injector device 10, such as sealing capabilities of the stopper 40. These enhanced characteristics may be produced, for example, by polishing or otherwise smoothing of the outer side 244 of the stopper 40 that ultimately contact the inner surface 124 of the barrel 20 and/or by transferring and depositing material of the outer side 244 of the stopper onto the inner surface 124 of the barrel 20 (e.g., when the tubular member 5300 in which the stopper 40 is modified is the barrel 20). In various embodiments, the processing provided by modification system 5230 can be performed after the injector device 10 is filled (e.g., with contents such as those previously described).

As referenced above, the modification system 5230 can be used for in-barrel processing of the stopper 20 and/or the modification system 5230 can be used to perform processing of the stopper 40 while the stopper 40 is within the vent tube 4264 during insertion of the stopper 40 into the barrel 20, such as that described in connection with FIGS. 10 to 14. By these embodiments the reductions of surface roughness of the stopper 40 are provided by the relative movement of the stopper 40 with respect to the vent tube 4264.

As shown, modification system 5230 includes a drive module 5234 and control module 5236 that can operate to process the outer side 40 of the stopper 40 and/or the inner surface 5310 of the tubular member 5300. Drive module 5234, which includes a shaft 5237 coupled to the stopper 40 in the illustrated embodiments (which may be the actuation mechanism 50 (FIGS. 1 and 2)), is controlled by the control module 5236 and produces relative motion between the tubular member 5300 and stopper 40. For example, the drive module 5234 can cause rotation 5240 of the stopper 40 with respect to the tubular member 5300 (e.g., by rotating the shaft 5237) in a direction generally perpendicular to the longitudinal axis X of the stopper 40 (FIGS. 3 and 4). Alternatively or in addition, the drive module 5234 can cause linear motion 5242 of the stopper 40 with respect to the tubular member 5300 (e.g., by moving the shaft 5237 with respect to the tubular member 5300) in a direction parallel to the longitudinal axis X of the stopper 40. The rotation 5240 and/or linear motion 5242 may be in a first or a second opposite direction, or reciprocal motion. In various embodiments, the rotation 5240 and/or linear motion 5242 is reciprocal motion in the first and second opposite directions, and may be periodic.

Alternatively or in addition, the drive module 5234 can cause rotation 5244 of the tubular member 5300 with respect to the stopper 40 by rotating the tubular member 5300 in a direction generally perpendicular to the longitudinal axis X of the stopper 40. Alternatively or in addition, the drive module 234 can cause linear motion 5246 of the tubular member 5300 with respect to the stopper 40 by moving the tubular member 5300 in a direction parallel to the longitudinal axis X of the stopper 40. For example, the modification system 5230 may include an actuation assembly 5238 (e.g., a roller, stage, or other actuatable member) secured to the tubular member 5300 and configured to move the tubular member 5300. The rotation 5244 and/or linear motion 5246 of the tubular member 5300 may be in a first or a second opposite direction, or reciprocal/oscillating motion. In various embodiments, the rotation 5244 and/or linear motion 5246 of the barrel 20 is reciprocal motion in the first and second opposite directions, and may be periodic. The rotation 5240, 5244 and/or linear motion 5242, 5246 between the tubular member 5300 and the stopper 40 may be vibratory motion.

In some embodiments, the modification system 5230 may produce relative rotation 5240 and/or 5244 and/or relative linear motion 5242 and/or 5246 of the tubular member 5300 with respect to the stopper 40 at any of a variety of combinations, rates, directions, and/or frequencies. In some embodiments, the relative motion produced by the modification system 5230 has greater amounts of rotation 5240 and/or 5244 than linear motion 5242 and/or 5246. The distances over which the inner surface 5310 of the tubular member 5300 moves with respect to the outer side 244 of the stopper 40 during this relative motion may be greater in the circumferential direction of the rotation 5240 and/or 5244 than in the direction parallel to the linear motion 5242 and/or 5246. In some embodiments, the rotation 5240 and/or 5244 is substantially greater than the linear motion 5242 and/or 5246 produced with the modification system 5230 during stopper processing/modification. In some embodiments, while the stopper 40 is being driven into the tubular member 5300 along a path parallel to the longitudinal axis X (e.g., without reciprocal motion), the stopper 40 may be rotated with respect to the tubular member 5300. For example, the stopper 40 may be rotated during longitudinal insertion into the vent tube 4264 and/or barrel 20 to modify the surface of the stopper 40 and/or the inner surfaces of the vent tube 4264 and/or barrel 20.

This surface modification, or processing in the tubular member 5300 (optionally, the vent tube 4264 and/or barrel 20), may help reduce roughness of the outer side 244 of the stopper 40 that ultimately engages the barrel 20. The roughness may be reduced in the circumferential direction and/or longitudinal direction. In some examples, surface roughness is decreased perpendicular to the direction of relative motion (e.g., longitudinal surface roughness may be decreased in the case of rotation 5240 and/or 5244 and circumferential surface roughness may be decreased in the case of linear motion 5242 and/or 5246.

In some embodiments, the processing of the stopper 40 with the modification system 5230 is performed at ambient temperatures (e.g., without heating or cooling by external sources), although modified temperatures (e.g., elevated temperatures) during treatment of the stopper 40 with the modification system 5230 are contemplated. The relative movement between the tubular member 5300 and the stopper 40 imparted by the modification system 5230 may cause heating of the tubular member 5300 and/or stopper 40, and such heating may reduce the roughness of the outer side 244 of the stopper 40 as described above.

The control module 5236 is configured to control operation of the system 1000. In various examples, the control module 5236 may include a power source (not shown), one or more microprocessors, one or more user input devices (e.g., keyboard), one or more display devices (e.g., monitor), and other features for controlling operation of the system 5230.

The power source may provide electrical power to the operative components of the control module 5236 and/or the other components of the system 5230, and may be any type of power source suitable for providing the desired performance and/or longevity requirements of the control module 5236 and/or system 5230. In various embodiments, the power source may include one or more batteries, which may be rechargeable (e.g., using an external energy source).

The control module 5236 may include, or be included in one or more Field Programmable Gate Arrays (FPGAs), one or more Programmable Logic Devices (PLDs), one or more Complex PLDs (CPLDs), one or more custom Application Specific Integrated Circuits (ASICs), one or more dedicated processors (e.g., microprocessors), one or more central processing units (CPUs), software, hardware, firmware, or any combination of these and/or other components. The control module 5236 may include a processing unit configured to communicate with memory to execute computer-executable instructions stored in the memory. Additionally, or alternatively, the control module 5236 may be configured to store information (e.g., sensed data) in the memory and/or access information (e.g., sensed data) from the memory.

In some embodiments, the memory includes computer-readable media in the form of volatile and/or nonvolatile memory and may be removable, nonremovable, or a combination thereof. Media examples include Random Access Memory (RAM); Read Only Memory (ROM); Electronically Erasable Programmable Read Only Memory (EEPROM); flash memory; optical or holographic media; magnetic cassettes, magnetic tape, magnetic disk storage or other magnetic storage devices; data transmissions; and/or any other medium that can be used to store information and can be accessed by a computing device such as, for example, quantum state memory, and/or the like. In embodiments, the memory stores computer-executable instructions for causing the processor to implement aspects of embodiments of system components discussed herein and/or to perform aspects of embodiments of methods and procedures discussed herein.

The computer-executable instructions may include, for example, computer code, digital signal processing, machine-useable instructions, and the like such as, for example, program components capable of being executed by one or more processors associated with the computing device. Program components may be programmed using any number of different programming environments, including various languages, development kits, frameworks, and/or the like. Some or all of the functionality contemplated herein may also, or alternatively, be implemented in hardware and/or firmware.

In some embodiments, the drive module 5234 is controlled by the control module 5236 and produces relative motion between the stopper components (e.g., body 240 and/or barrier 242) and the tubular member 5300 during surface modification. As referenced, the drive module 5234 can cause rotation of one or more of the stopper components (e.g., body 240 and/or barrier 242) and/or the tubular member 5300. And, the drive module 5234 may additionally or alternatively produce axial movement of the stopper components (e.g., the body 240 and/or barrier 242) and/or the tubular member 5300. The drive module 5234 may include drive motors, sensors, control circuits, drive shafts, turn tables, and/or a variety of additional or alternative components for achieving the desired, relative motion.

It is contemplated that the heating produced during processing with the modification system 5230 may cause the temperature of the material of the stopper 40 (e.g., at outer side 244) to increase sufficiently to encourage polymeric movement at the outer side 244 and reduce wrinkles, scratches, debris, or other unwanted surface defects that may contribute to surface roughness and/or a likelihood of seal defects at the interface between the stopper 40 and the barrel 20. The temperature increase induced by the relative movement may be below the melting temperature, above the melting temperature, or otherwise at a desired temperature that, when coupled with the mechanical engagement / manipulation caused by the relative movement, the surface defects / roughness are reduced as desired. In various examples, the relative motion between the stopper 40 and the tubular member 5300 results in localized heating one or more of the ribs 300.

Although stopper 40 is shown generically, the modifications may only be applied to the ribs 300 or micro ribs 400 rather than to the entire outer side 244. In fact, the surface modifications may apply to only one rib 300 or even one micro rib 400. Amount or degree of modification may be impacted by initial geometry of the stopper 40. For example, those ribs 300 with a larger interference fit with the barrel 20 will naturally cause a higher degree of friction and energy. In some examples, the second or third rib 300 may be configured to engage with the barrel 20 in such a manner to promote the surface modification. By encouraging modification at one or more intermediate ribs, rather than a leading rib, it may help avoid issues of unwanted particulate creation and deposition into the barrel contents.

It is also contemplated that the stopper 40 processing in the tubular member 5300 may approach the reduction of roughness of the outer side 244 without producing substantial amounts of unwanted particulate / contaminants. Particulates that are formed as part of the surface modification or otherwise may coalesce or be encapsulated into deformed or melted material of the stopper 40.

It is also contemplated that during in-barrel processing using the modification system 5230, surface defects in the barrel 20 may be reduced to enhance seal and/or sliding performance. For example, the relative motion between the barrel 20 and stopper 40 may induce smearing or transfer of material from the outer side 244 of the stopper 40 onto the inner surface 124 of the barrel 20. Such transfer of material may create a seal line (e.g., in the form of a friction weld) or may simply fill in surface irregularities (e.g., scratches) in the barrel 20 to encourage a more reliable seal with the stopper 40. Such transfer of material from the stopper 40 to the barrel 20 to help reduce the impact of imperfections in the barrel surface (inner surface 124) and/or generate a configuration of the seal interface 702 in which the seal interface 702 includes the same or similar materials. For example, a PTFE-to-PTFE (or an ePTFE-to-eTPFE interface) formed by transferred material on the barrel 20 and the barrier material 242 on the stopper 40, may have enhanced sealing and/or sliding performance. It is contemplated that such a configuration for the seal interface 702 may have a lower likelihood of leaking based upon the surface energies between the two similar materials.

Various aspects of this disclosure relate to methods for manufacturing injector device 10, 100 including modifying the stopper 40 via relative movement, or motion. In various examples, modifying the stopper 40 includes modifying the outer side 244 of the stopper 40, such as by melting or otherwise inducing polymeric movement of a portion of the stopper 40, which may ultimately improve seal integrity of the stopper 40 with the barrel 20. It is contemplated that seal integrity may be improved by one or more of reducing surface roughness of the outer side of the stopper 40, reducing wrinkling in the outer side 244 of the stopper 40, forming a seal line between the outer side 244 of the stopper 40 and the inner surface 124 of the barrel 20, and/or decreasing one or more leak paths between the stopper 40 and the barrel 20, for example. In some examples, modifying the stopper includes modifying an activatable layer of the stopper 40 by directing energy through the wall 118 of the barrel 20 to the activatable layer.

FIGS. 16A to 19B are schematic illustrations showing potential benefits of such manufacturing techniques at a seal interface 702 corresponding to the area "A" of the stopper 40 shown in FIG. 5. FIGS. 16A to 18B indicate potential defects 700 in the form of surface irregularities in the barrel 20, as the inner surface 124 of the barrel 20 is not perfectly smooth. Such defects 700 may be scratches, irregularities, voids or pores, or other features. As shown in FIG. 16A, it is contemplated that in various embodiments following surface modification of the stopper 40 by inducing relative movement between the barrel 20 and the stopper 40, the barrier 242 of the stopper 40 may conforms more closely to the barrel 20 by accommodating, or better filling in, the defects 700 (e.g., at locations where the stopper 40 engages the barrel 20, such as proximate the macro and/or micro features 400. FIG. 16B is meant to illustrate this concept, where the defects 700 are shown at least partially filled with material from the outer side 244 of the stopper 40, and specifically the barrier 242 of the stopper 40.

As previously referenced, the outer side 244 of the stopper 40 may include a polymeric material (e.g., FEP, ePTFE, PTFE, and/or another polymeric material described herein) that forms a seal interface 702 with the barrel 20, and modifying the stopper 40 includes inducing polymeric movement of the polymeric material at the seal interface 702 via the relative movement of the components. As shown in FIG. 16A, prior to such filling in, or accommodation of the defects 700, there may be a space 710, or potential leak path 710, between the stopper 40 (the barrier 242) and the barrel 20. And, after surface modification, the space 710, or potential leak path 710, is more effective sealed, or closed at the seal interface 702. This, in turn, may result in a relatively more secure, or stable seal at the seal interface 702 (e.g., proximate one or more of the macro features 300 or micro features 400.

Also, as shown in FIG. 16A, the seal interface 702 between the outer side 244 of the stopper 40 and the barrel 20 may include particulate 800 (e.g., debris) introduced into the seal interface 702. Such particulate may include portions of the stopper 40 or barrel 20 that have broken off or were loosened during manufacturing, or other foreign matter. As shown in FIG. 16B, during in-barrel processing, such particulate may be reflowed or coalesced into the seal interface 702. Clearly, a reduction in such particulate would be desirable, particularly in pharmaceutical applications where contamination of the barrel contents is particularly undesirable.

Also, as shown in FIG. 16A and as previously referenced with respect to FIGS. 3 and 4, the surface of the stopper 40, and in particular the barrier 242, may include one or more wrinkles or surface defects 900. Such surface defects 900 may be created during insertion of the stopper 40 into the barrel 20, during manufacture, or otherwise. Modification of the syringe stopper 40 through induced relative movement between the stopper and the tubular member 5300 (e.g., the barrel 20 as shown in FIGS. 16A and 16B) may result in polymeric movement of the material of the barrier 242, thereby smoothing out the surface defects or wrinkles. This may, in turn, help bring the outer side 244 of the stopper 40 into closer conformity with the inner surface 124 of the barrel 20 (either after the stopper 40 is assembled into the barrel 20 or after the stopper 40 is assembled into the barrel 20). This, in turn, can be said to reduce the roughness of the outer surface 244 of the stopper 40. As previously mentioned, the surface modification could be applied to the stopper in a circumferential pattern (e.g., through continuous, oscillating, or other relative rotation) to help enhance seal integrity.

FIGS. 17A and 17B show a similar effect, where the surface of the barrier 242 is reflowed, or mobilized using the energy (e.g., frictional energy) to fill in defects (e.g., scratches or grooves) in the inner surface 124 of the barrel 20. As shown in FIG. 17A, there is a space or potential leak path 710 between the barrel 20 and the stopper 40. By inducing relative movement, and mobilization of the surface of the barrier 242, the potential leak path 710 is filled, enhancing overall seal integrity. And, similarly to FIGS. 16A and 16B, the relative movement can be applied in a desired pattern (e.g., circumferential and/or longitudinal) to create a desired effect.

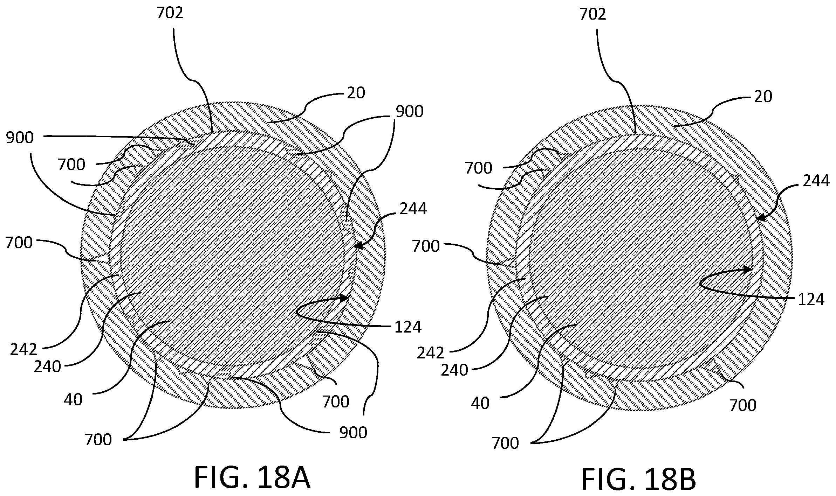

FIGS. 18A and 18B illustrate similar principals to FIGS. 16A to 17B regarding the application of energy to the stopper 40 to cause surface modification of the stopper 40. FIGS. 18A and 18B are illustrations along a transverse cross-section through the injector device 10. FIGS. 18A and 18B may be representative of a transverse cross-section of the injector device 10 including the area "A" designated in FIG. 5, for example at a seal interface 702 between the stopper 40 and barrel 20. FIG. 18A depicts the barrel 20, and specifically the defects 700 in the form of surface irregularities (e.g., scratches) about the circumference of the inner surface 124 of the barrel 20 from a longitudinal view. Also shown are wrinkles or surface defects 900 in the outer side 244 about the circumference of the stopper 40.

FIG. 18B illustrates a contemplated effect on the seal interface 702 following surface modification. As shown, induced polymeric movement of the stopper 40 (e.g., of the barrier 242) may result in filling of the defects 700 in the barrel 20, smoothing of the wrinkles or surface defects in the stopper 40, enhancement of the seal interface 702, and creation of a circumferential seal line corresponding to the seal interface 702, for example.

In view of the foregoing, various methods for manufacturing the injector device 10, 100 include in-barrel processing of the stopper 40. An example method may include positioning the stopper 40 in vent tube 4264, inserting the vent tube 4264 into the barrel 20, delivering the stopper 40 from the vent tube 4264 into the barrel 20 such that the outer side 244 is engaged with the inner surface 124 of the barrel 20 to define seal interface 702 between the outer side 244 of the stopper 40 and the inner surface 124 of the barrel 20, and inducing relative motion between the stopper 40 and the barrel 20 to enhance the seal interface 702 between the outer side 244 of the stopper 40 and the inner surface 124 of the barrel 20. As previously referenced, the relative motion may include a rotational component, longitudinal component, or combinations thereof. As discussed, after the stopper 40 is delivered from the vent tube 4264 into the barrel 20, the outer side 244 of the stopper 40 may include wrinkling at the seal interface 702, and enhancing the seal interface between the outer side 244 of the stopper 40 and the inner surface 124 of the barrel 20 includes reducing wrinkling at the seal interface 702. Enhancing the seal interface 702 between the outer side of the stopper 40 and the inner surface 124 of the barrel 20 includes transferring material from the outer side 244 of the stopper 40 to the inner side 124 of the barrel 20. One or more of the ribs 300 may define the seal interface 702.

As previously mentioned, a transfer of material from the stopper 40 to the barrel 20 may occur to help reduce the impact of imperfections in the barrel surface (inner surface 124) and/or generate a configuration of the seal interface 702 in which the seal interface 702 includes the same or similar materials. For example, a PTFE-to-PTFE (or an ePTFE-to-eTPFE interface) formed by transferred material on the barrel 20 and the barrier material 242 on the stopper 40, may have enhanced sealing and/or sliding performance. It is contemplated that such a configuration for the seal interface 702 may have a lower likelihood of leaking based upon the surface energies between the two similar materials.

U.S. Patent 5,772,755 by Applicant W. L. Gore & Associates, Inc. provides evidence of the feasibility of this mechanism of transfer between the barrier 242 and barrel 20 (e.g., made of borosilicate glass). For example, that patent reference describes the coating of a glass plate to produce oriented PTFE. Specifically, the glass plate was placed on a platform and heated by radiant heat to a temperature of 200 degree C. A tape of PTFE was prepared by lubrication (paste) extrusion of coagulated dispersion type PTFE, evaporating the lubricate and stretching the extruded tape at 2:1 to make the tape compliant. This tape was wrapped around a heatable bar about 14 inches long. The wrapped bar was then heated to about 300 degree C and then the bar dragged over the glass substrate with adjustable force. Multiple passes were made to ensure complete coverage of the glass surface. By dragging the PTFE bar, PTFE was deposited on the surface of the glass in aligned rows of PTFE chains.

It is contemplated that, similar to the mechanism described above, relative motion between the barrier 242 and barrel 20 will result in some heating and transfer of material (e.g., PTFE or ePTFE) with an orientation corresponding to the direction of relative movement (e.g., circumferential, longitudinal, or combinations thereof depending upon the particular implementation.

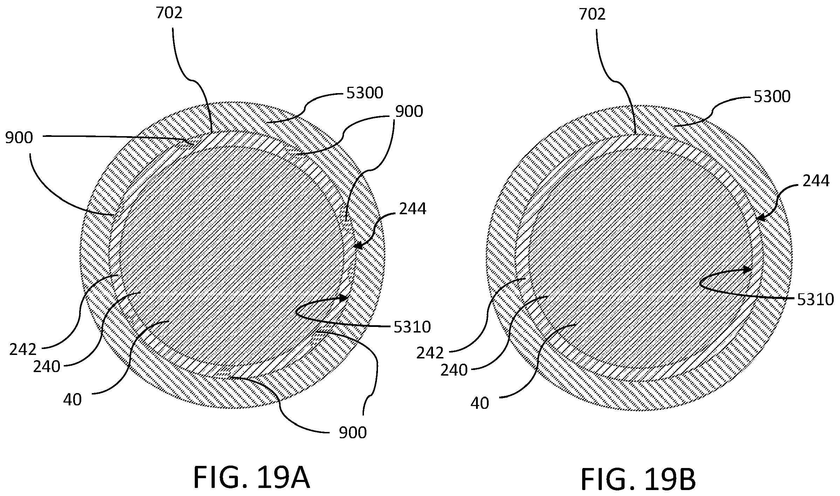

Thus, in various examples, the injector device 10, 100 includes a barrel and a stopper defining a seal interface, the barrel having deposited material corresponding to the material of the barrier, the deposited material having a directional orientation. The directional orientation of the material may be defined by rows of PTFE chains aligned in a common direction, for example. The common direction may be circumferential, longitudinal, or combinations thereof. FIGS. 19A and 19B illustrate similar principals as FIGS. 18A and 18B, but with respect to the more general tubular member 5300, which may be the vent tube 4264 or other tubular member. As previously referenced, the outer side 244 of the stopper 40 can include a polymeric material (e.g., FEP, ePTFE, PTFE, and/or another polymeric material described herein). And, modifying the stopper 40 includes inducing polymeric movement of the polymeric material at the interface between the stopper 40 and the tubular member 5300. It is contemplated that the surface of the stopper 40, and in particular the barrier 242, may include one or more wrinkles or surface defects 900. Again, such surface defects 900 may be created during manufacture, or otherwise. Modification of the syringe stopper 40 through induced relative movement between the stopper and the tubular member 5300 may result in polymeric movement of the material of the barrier 242, thereby smoothing out the surface defects or wrinkles. This may, in turn, help bring the outer side 244 of the stopper 40 into closer conformity with the inner surface 124 of the barrel 20 (either after the stopper 40 is assembled into the barrel 20 or after the stopper 40 is assembled into the barrel 20). This, in turn, may result in a reduction in the roughness of the outer surface 244 of the stopper 40. As previously mentioned, the surface modification could be applied to the stopper in a circumferential pattern (e.g., through continuous, oscillating, or other relative rotation) to help enhance seal integrity.

### Example Material Sets

The barrel 20 may be formed of a substantially rigid or hard material, such as a glass material (e.g., borosilicate glass), a ceramic material, one or more polymeric materials (e.g., polypropylene, polyethylene, and copolymers thereof), a metallic material, or a plastic material (e.g., cyclic olefin polymers (COC) and cyclic olefin copolymers (COP), and combinations thereof. It is to be appreciated that barrels formed of materials that are not inherently hydrophobic (e.g. a glass barrel) may be coated or otherwise treated so as to be rendered hydrophobic. In some embodiments, the barrels 20 has a hydrophobic interior wall characterized by the absence of a lubricant such as, but not limited to, silicone or silicone oil. As used herein, the term "hydrophobic interior wall" refers to the interior surface of a barrel that is free or substantially free (i.e., has an unquantifiable or trace amount) of silicone oil. In addition, the hydrophobic surface of the barrel 20 also has a contact angle of deionized water on a flat surface of the material greater than 90°, indicating a hydrophobic surface. In some embodiments, the water contact angle is from about 90° to about 180° or from about 96° to about 180°, from about 96° to about 130, or from about 96° to about 120°.

In some embodiments, the body 240 of the stopper 40 is formed of a suitable elastomer, such as a rubber material. Examples of suitable rubber materials include synthetic rubbers, thermoplastic elastomers, and materials prepared by blending synthetic rubbers and the thermoplastic elastomers. The material may be rubbers constructed from butyl, bromobutyl, or chlorobutyl, a halogenated butyl rubber, a styrene butadiene rubber, a butadiene rubber, an epichlorohydrin rubber, a neoprene rubber, an ethylene propylene rubber, silicone, nitrile, styrene butadiene, polychloroprene, ethylene propylene diene, fluoroelastomers, thermoplastic elastomers (TPE), thermoplastic vulcanizates (TPV), materials sold under the trade name VITON^{®}, and combinations and blends thereof. In some embodiments, the body 240 may have an initial modulus (small strain) of between about 2.5 MPa to about 5 MPa, or between about 3 MPa to about 4 MPa. In some embodiments, the initial modulus is about 3.5 MPa, although a variety of values are contemplated.

As previously referenced, portions of the barrier 242 (e.g., layers or zones) may be configured to be more activatable, or reactive, to an energy source than other layers or zones of the barrier 242. For example, in the case of laser or other optical energy sources, the reactivity or ability to be activated, may be adjusted by modifying material thickness, pigmentation, density/open space/air content, chemical / material composition, and others. In the case of radiofrequency (RF), electrical and electromagnetic energy sources, the barrier 242 may be adjusted to include pigments or other fillers, such as metallics (e.g., iron, platinum, or others), that are more reactive to such energy. In the case of microwave energy sources, metallics, water, or other materials may be implemented. And, in the case of ultraviolet (UV) energy cross-linking agents (acrylates that would cross-link and increase density / stiffness) or other materials that absorb UV energy may be incorporated.

Examples suitable materials for one or more layers of the barrier 242 of the stopper include films of ultrahigh molecular weight polyethylenes and fluororesins. The barrier 242 may include a fluoropolymer film, such as a polytetrafluoroethylene (PTFE) film or a densified expanded polytetrafluoroethylene (ePTFE) film. Film and film composites including PTFE or ePTFE can help provide thin and strong barrier layers to leachables and extractables that may be present in the underlying elastomer and might otherwise contaminate the therapeutic substance in the barrel.

Some specific examples of suitable materials of the barrier 242 include, but are not limited to, the following: (1) A PTFE (polytetrafluoroethylene) homopolymer film produced by the skiving method (e.g., VALFLON (trade name) available from Nippon Valqua Industries, Ltd.); (2) A modified PTFE (a copolymer of a tetrafluoroethylene monomer and several percents of a perfluoroalkoxide monomer) film produced by the skiving method (e.g., NEW VALFLON (trade name) available from Nippon Valqua Industries, Ltd.); and (3) An ultrahigh molecular weight polyethylene film produced by the skiving method (e.g., NEW LIGHT NL-W (trade name) available from Saxin Corporation).

As indicated, the barrier 242 may be a composite or laminate material, or otherwise include a multi-component (e.g., multi-layer) barrier. Other suitable fluoropolymers for use in or as the barrier 242 include, but are not limited to, fluorinated ethylene propylene (FEP), polyvinylidene fluoride, polyvinylfluoride, perfluoropropylvinylether, perfluoroalkoxy polymers, tetrafluoroethylene (TFE), Parylene AF-4, Parylene VT-4, and copolymers and combinations thereof. Non-fluoropolymers such as, but not limited to, polyethylene, polypropylene, Parylene C, and Parylene N may also or alternatively be used to form the barrier 242.

A densified ePTFE film for the barrier 242 may be prepared in the manner described in U.S. Pat. 7,521,010 to Kennedy, et al., U.S. Pat. No. 6,030,694 to Dolan et al., U.S. Pat. No. 5,792,525 to Fuhr et al., or U.S. Pat. No. 5,374,473 to Knox et al. Expanded copolymers of PTFE may also be used for the barrier 242, such as those described in U.S. Pat. No. 5,708,044 to Branca, U.S. Pat. No. 6,541,589 to Baillie, U.S. Pat. No. 7,531,611 to Sabol et al., U.S. Pat. No. 8,637,144 to Ford, and U.S. Pat. No. 9,139,669 to Xu et al., particularly if they are densified.

In one or more embodiment, the barrier 242 may include, or be formed of, one or more of the following materials: ultra-high molecular weight polyethylene as taught in U.S. Pat. No. 9,926,416 to Sbriglia; polyparaxylylene as taught in U.S. Patent Publication No. 2016/0032069 to Sbriglia; polylactic acid as taught in U.S. Pat. No. 9,732,184 to Sbriglia, et al.; and/or VDF-co-(TFE or TrFE) polymers as taught in U.S. Pat. No. 9,441,088 to Sbriglia.

The barrier 242 may also include an expanded polymeric material including a functional tetrafluoroethylene (TFE) copolymer material having a microstructure characterized by nodes interconnected by fibrils, where the functional TFE copolymer material includes a functional copolymer of TFE and PSVE (perfluorosulfonyl vinyl ether), or TFE with another suitable functional monomer, such as, but not limited to, vinylidene fluoride (VDF), vinyl acetate, or vinyl alcohol. The functional TFE copolymer material may be prepared, for example, according to the methods described in U.S. Pat. No. 9,139,669 to Xu et al. or U.S. Pat. No. 8,658,707 to Xu et al.

In some embodiments, the barrier 242 may be formed of a composite fluoropolymer or non-fluoropolymer material having a barrier layer and a tie layer such as is described in U.S. Patent Publication No. 2016/0022918 to Gunzel. It is to be noted that, as used herein, the term "tie layer" may include fluoropolymer and/or non-fluoropolymer materials. The tie layer can include, or be formed of, expanded polytetrafluoroethylene or other porous expanded fluoropolymers (for example, an ePTFE as taught in U.S. Pat. No. 6,541,589 to Baille). Alternatively, the tie layer may be formed of, or include, non-fluoropolymer materials. Non-limiting examples of suitable non-fluoropolymer materials for use in or as the tie layer include non-fluoropolymer membranes, non-fluoropolymer microporous membranes, non-woven materials (e.g., spunbonded, melt blown fibrous materials, electrospun nanofibers), polyvinylidene difluoride (PVDF), nanofibers, polysulfones, polyethersulfones, polyarlysolfones, polyether ether ketone (PEEK), polyethylenes, polypropylenes, and polyimides.

In some embodiments, the barrier 242 can be made by forming a thin densified composite comprising a porous ePTFE layer and a thermoplastic barrier layer. In this aspect, a thermoplastic having a surface with a low coefficient of friction is preferred. Accordingly, fluoropolymer-based thermoplastics such as fluorinated ethylene propylene (FEP), perfluoroalkoxy (PFA), a polymer of tetrafluoroethylenes, hexafluoropropylene and vinylindene fluoride (THV) may be applicable. A barrier according to this aspect may be an FEP/ePTFE laminate obtained by following the process taught in WO 94/13469 to Bacino. The barrier may be formed at process temperatures above the softening temperature or even above the melt of the FEP film in a female cavity mold.

In some embodiments, the barrier 242 may comprise a composite of a densified ePTFE film and a thin layer of porous ePTFE bonded to the barrier layer film. The densified ePTFE film may be obtained as described in U.S. Pat. No. 7,521,010 to Kennedy et al. The ePTFE/densified ePTFE composite may be combined in the manner described in U.S. Pat. No. 6,030,694 to Dolan, et al. In this embodiment, the composite material comprises a layer of densified ePTFE film and a porous ePTFE layer.

In some embodiments, the barrier 242 includes a composite material having at least three layers, namely, a densified expanded fluoropolymer layer, a barrier melt fluoropolymer layer, and a porous layer. The densified expanded fluoropolymer layer may include or be formed of a densified ePTFE. The barrier melt fluoropolymer layer may include a fluoropolymer such as a densified expanded fluoropolymer, polytetrafluoroethylene (PTFE), expanded polytetrafluorethylene (ePTFE), densified expanded polytetrafluoroethylene, fluorinated ethylene propylene (FEP), polyvinylidene fluoride, polyvinylfluoride, perfluoropropylvinylether, perfluoroalkoxy polymers, and copolymers and combinations thereof. Non-limiting examples of non-fluoropolymers that may be utilized in the barrier melt layer include polyethylene and polypropylene. The porous layer may include or be formed of ePTFE or other porous expanded fluoropolymers. The laminate layers having the densified expanded fluoropolymer layer, the barrier melt fluoropolymer layer and the porous layer may be constructed by coating or otherwise depositing the densified expanded fluoropolymer onto the porous layer to create the composite material. In one non-limiting embodiment, the laminate layer is formed of a densified fluoropolymer (e.g., densified ePTFE), a thermoplastic adhesive (e.g., FEP), and a porous fluoropolymer (e.g., ePTFE).

It is to be appreciated that the stopper 40 may include various degrees of penetration of either the material of the body 240 into the materials of the barrier 242 or vice versa, including those described in U.S. Pat. No. 8,722,178 to Ashmead, et al., U.S. Pat. No. 9,597,458 to Ashmead, et al., and U.S. Patent Publication No. 2016/0022918 to Gunzel. It is also to be appreciated that there are many variations of the processes described herein that could be utilized for forming the stopper 40.

### Examples of Therapeutic Substances

The syringes, tip caps, and other embodiments of the present disclosure may be used in combination with different therapeutic compounds including, but not limited to, drugs and biologics such as Coagulation Factors, Cytokines, Epigenetic protein families, Growth Factors, Hormones, Peptides, Signal Transduction molecules, and mutations thereof; also including Amino Acids, Vaccines and/or combinations thereof. Therapeutic compounds further include antibodies, antisense, RNA interference made to the above biologics and their target receptors and mutations of thereof. Additional therapeutic compounds include Gene Therapy, Primary and Embryonic Stem Cells. Also included in the therapeutic compounds are antibodies, antisense, RNA interference to Protein Kinases, Esterases, Phosphatases, Ion channels, Proteases, structural proteins, membrane transport proteins, nuclear hormone receptors and/or combinations thereof. Additionally, it is to be understood that at least one of the therapeutic compounds identified herein used in the instant disclosure, also two or more therapeutic compounds listed in this application are considered to be within the purview of the present disclosure.

Examples of Coagulation Factors include, but are not limited to: Fibrinogen, Prothrombin, Factor I, Factor V, Factor X, Factor VII, Factor VIII, Factor XI, Factor XIII, Protein C, Platelets, Thromboplastin, and Co-factor of VIIa.

Examples of Cytokines include, but are not limited to: Lymphokines, Interleukins, Chemokines, Monokines, Interferons, and Colony stimulating factors.

Examples of Epigenetic protein families include, but are not limited to: ATPase family AAA domain-containing protein 2 (ATAD2A), ATPase family-AAA domain containing 2B (ATAD2B), ATPase family AAA domain containing-2B (ATAD2B), bromodomain adjacent to zinc finger domain-1A (BAZ1A), bromodomain adjacent to zinc finger domain-1B (BAZ1B), bromodomain adjacent to zinc finger domain-2A (BAZ2A), bromodomain adjacent to zinc finger domain-2A (BAZ2A), bromodomain adjacent to zinc finger domain-2B (BAZ2B), bromodomain-containing protein 1 (BRD1), Bromodomain containing protein 2-1st bromodomain (BRD2), Bromodomain containing protein 2-1st & 2nd bromodomains (BRD2), bromodomain-containing protein 2 isoform 1-bromodomain 2 (BRD2(2)), bromodomain-containing protein 3-bromodomain 1 (BRD3(1)), Bromodomain-containing protein 3-1st bromodomain (BRD3), Bromodomain-containing protein 3-1st & 2nd bromodomains (BRD3), bromodomain-containing protein 3-bromodomain 2 (BRD3(2)), Bromodomain containing protein 4-1st bromodomain (BRD4), bromodomain-containing protein 4 isoform long-bromodomains 1 and 2 (BRD4(1-2)), bromodomain-containing protein 4 isoform long-bromodomain 2 (BRD4(2)), bromodomain-containing protein 4 isoform short (BRD4(full-length-short-iso.)), Bromodomain containing protein 7 (BRD7), bromodomain containing 8-bromodomain 1 (BRD8(1)), bromodomain containing 8-bromodomain 2 (BRD8(2)), bromodomain-containing protein 9 isoform 1 (BRD9), Bromodomain containing testis-specific-1st bromodomain (BRDT), Bromodomain containing testis-specific-1st & 2nd bromodomains (BRDT), bromodomain testis-specific protein isoform b-bromodomain 2 (BRDT(2)), bromodomain and PHD finger containing-1 (BRPF1), bromodomain and PHD finger containing-3 (BRPF3), bromodomain and PHD finger containing-3 (BRPF3), Bromodomain and WD repeat-containing 3-2nd bromodomain (BRWD3(2)), Cat eye syndrome critical region protein 2 (CECR2), CREB binding protein (CREBBP), E1A binding protein p300 (EP300), EP300 (EP300), nucleosome-remodeling factor subunit BPTF isoform 1 (FALZ), Nucleosome-remodeling factor subunit BPT (FALZ), Euchromatic histone-lysine N-methyltransferase 2 (EHMT2), Histone Acetyltransferase-KAT2A (GCN5L2), Euchromatic histone-lysine N-methyltransferase 1 (EHMT1), Histone-lysine N-methyltransferase MLL (MLL), Polybromo 1-1st bromodomain (PB1(1)), Polybromo 1-2nd bromodomain (PB1(2)), polybromo 1-bromodomain 2 (PBRM1(2)), polybromo 1-bromodomain 5 (PBRM1(5)), Histone acetyltransferase KAT2B (PCAF), PH-interacting protein-1st bromodomain (PHIP(1)), PH-interacting protein-2nd bromodomain (PHIP(2)), Protein kinase C-binding protein 1 (PRKCBP1), Protein arginine N-methyltransferase 3 (PRMT3), SWI/SNF related-matrix associated-actin dependent regulator of chromatin-subfamily a-member 2 (SMARCA2), SWI/SNF related-matrix associated-actin dependent regulator of chromatin-subfamily a-member 4 (SMARCA4), Nuclear body protein-SP110 (SP110), Nuclear body protein-SP140 (SP140), Transcription initiation factor TFIID subunit 1 (TAF1(1-2)), TAF1 RNA polymerase II-TATA box binding protein (TBP)-associated factor-250 kDa-bromodomain 2 (TAF1(2)), Transcription initiation factor TFIID subunit 1-like-1 st bromodomain (TAF1L(1)), Transcription initiation factor TFIID subunit 1-like-2nd bromodomain (TAF1L(2)), tripartite motif containing 24 (TRIM24(Bromo.)), tripartite motif containing 24 (TRIM24(PHD-Bromo.)), E3 ubiquitin-protein ligase TRIM33 (TRIM33), tripartite motif containing 33 (TRIM33(PHD-Bromo.)), WD repeat 9-1st bromodomain (WDR9(1)), and WD repeat 9-2nd bromodomain (WDR9(2)).

Examples of growth factors include, but are not limited to: nerve growth factor (NGF), vascular endothelial growth factor (VEGF), platelet-derived growth factor (PDGF), C-fos-induced growth factor (FIGF), platelet-activating factor (PAF), transforming growth factor beta (TGF-β), bone morphogenetic proteins (BMPs), Activin, inhibin, fibroblast growth factors (FGFs), granulocyte-colony stimulating factor (G-CSF), granulocyte-macrophage colony stimulating factor (GM-CSF), glial cell line-derived neurotrophic factor (GDNF), growth differentiation factor-9 (GDF9), epidermal growth factor (EGF), transforming growth factor-α (TGF-α), growth factor (KGF), migration-stimulating factor (MSF), hepatocyte growth factor-like protein (HGFLP), hepatocyte growth factor (HGF), hepatoma-derived growth factor (HDGF), and Insulin-like growth factors.

Examples of Hormones include, but are not limited to: Amino acid derived (such as melatonin and thyroxine), Thyrotropin-releasing hormone, Vasopressin, Insulin, Growth Hormones, Glycoprotein Hormones, Luteinizing Hormone, Follicle-stimulating Hormone, Thyroid-stimulating hormone, Eicosanoids, Arachidonic acid, Lipoxins, Prostaglandins, Steroid, Estrogens, Testosterone, Cortisol, and Progestogens.

Examples of Proteins and Peptides and Signal Transduction molecules include, but are not limited to: Ataxia Telangiectasia Mutated, Tumor Protein p53, Checkpoint kinase 2, breast cancer susceptibility protein, Double-strand break repair protein, DNA repair protein RAD50, Nibrin, p53-binding protein, Mediator of DNA damage checkpoint protein, H2A histone family member X, Microcephalin, C-terminal-binding protein 1, Structural maintenance of chromosomes protein 1A, Cell division cycle 25 homolog A (CDC25A), forkhead box O3 (forkhead box O3), nuclear factor of kappa light polypeptide gene enhancer in B-cells inhibitor, alpha (NFKBIA), nuclear factor (erythroid-derived 2)-like 2 (NFE2L2), Natriuretic peptide receptor A (NPR1), Tumor necrosis factor receptor superfamily, member 11a (TNFRSF11A), v-rel reticuloendotheliosis viral oncogene homolog A (avian) (RELA), Sterol regulatory element binding transcription factor 2 (SREBF2), CREB regulated transcription coactivator 1 (CRTC1), CREB regulated transcription coactivator 2 (CRTC2), X-box binding protein 1 (XBP1), and Catenin beta 1 (cadherin-associated protein or CTNNB1).

Examples of G Protein-Coupled Receptors (GPCR) include, but are not limited to: Adenosine receptor family, Adrenergic receptor family, Angiotensin II receptor, Apelin receptor, Vasopressin receptor family, Brain-specific angiogenesis inhibitor family, Bradykinin receptor family, Bombesin receptor family, Complement component 3a receptor 1, Complement component 5a receptor 1, Calcitonin receptor family, Calcitonin receptor-like family, Calcium-sensing receptor, Cholecystokinin A receptor (CCK1), Cholecystokinin B receptor (CCK2), Chemokine (C-C motif) receptor family, Sphingosine 1-phosphate receptor family, Succinic receptor, Cholinergic receptor family. Chemokine-like receptor family, Cannabinoid receptor family, Corticotropin releasing hormone receptor family, prostaglandin D2 receptor, Chemokine C-X3-C receptor family, Chemokine (C-X-C motif) receptor family, Burkitt lymphoma receptor, Chemokine (C-X-C motif) receptor family, Cysteinyl leukotriene receptor 2 (CYSLT2), chemokine receptor (FY), Dopamine receptor family, G protein-coupled receptor 183 (GPR183), Lysophosphatidic acid receptor family, Endothelin receptor family, Coagulation factor II (thrombin) receptor family, Free fatty acid receptor family, Formylpeptide receptor family, Follicle stimulating hormone receptor (FSHR), gamma-aminobutyric acid (GABA) B receptor, Galanin receptor family, Glucagon receptor, Growth hormone releasing hormone receptor (GHRH), Ghrelin receptor (ghrelin), Growth hormone secretagogue receptor 1b (GHSR1b), Gastric inhibitory polypeptide receptor (GIP), Glucagon-like peptide receptor family, Gonadotropin-releasing hormone receptor (GnRH), pyroglutamylated RFamide peptide receptor (QRFPR), G protein-coupled bile acid receptor 1 (GPBA), Hydroxycarboxylic acid receptor family, Lysophosphatidic acid receptor 4 (LPA4) Lysophosphatidic acid receptor 5 (GPR92), G protein-coupled receptor 79 pseudogene (GPR79), Hydroxycarboxylic acid receptor 1 (HCA1), G-protein coupled receptor (C5L2, FFA4, FFA4, FFA4, GPER, GPR1, GPR101, GPR107, GPR119, GPR12, GPR123, GPR132, GPR135, GPR139, GPR141, GPR142, GPR143, GPR146, GPR148, GPR149, GPR15, GPR150, GPR151, GPR152, GPR157, GPR161, GPR162, GPR17, GPR171, GPR173, GPR176, GPR18, GPR182, GPR20, GPR22, GPR25, GPR26, GPR27, GPR3, GPR31, GPR32, GPR35, GPR37L1, GPR39, GPR4, GPR45, GPR50, GPR52, GPR55, GPR6, GPR61, GPR65, GPR75, GPR78, GPR83, GPR84, GPR85, GPR88, GPR97, TM7SF1), Metabotropic glutamate receptor family, Gastrin releasing peptide receptor (BB2), Orexin receptor family, Histamine receptor family, 5-hydroxytryptamine receptor family, KISS1-derived peptide receptor (kisspeptin), Leucine-rich repeat-containing G protein-coupled receptor family, horiogonadotropin receptor (LH), Leukotriene B4 receptor (BLT1), Adenylate Cyclase Activating Polypeptide 1 Receptor 1 (mPAC1), Motilin receptor, Melanocortin receptor family, Melanin concentrating hormone receptor 1 (MCH1), Neuropeptide Y1 receptor (Y1), Neuropeptide Y2 receptor (NPY2R), Opioid receptor family, Oxytocin receptor (OT), P2Y Purinoceptor 12 (mP2Y12), P2Y Purinoceptor 6 (P2Y6), Pancreatic polypeptide receptor family, Platelet-activating factor receptor family, Prostaglandin E receptor family, Prostanoid IP1 receptor (IP1), MAS-related GPR, member family, Rhodopsin (Rhodopsin), Relaxin family peptide receptor family, Somatostatin receptor family, Tachykinin receptor family, Melatonin receptor family, Urotensin receptor family, Vasoactive intestinal peptide receptor 1 (mVPAC1), Neuromedin B Receptor (BB1), Neuromedin U receptor 1 (NMU1), Neuropeptides B/W receptor family, Neuropeptide FF receptor 1 (NPFF1), neuropeptide S receptor 1 (NPS receptor), Neuropeptide Y receptor family, Neurotensin receptor 1 (NTS1), Opsin 5 (OPN5), Opioid receptor-like receptor (NOP), Oxoeicosanoid (OXE) receptor 1 (OXE), Oxoglutarate (alpha-ketoglutarate) receptor 1 (OXGR1), Purinergic receptor family, Pyrimidinergic receptor family, Prolactin releasing hormone receptor (PRRP), Prokineticin receptor family, Platelet activating receptor (PAF), Prostaglandin F receptor family, Prostaglandin 12 (prostacyclin) receptor family, Parathyroid hormone receptor family, muscarinic acetylcholine receptors (such as rM4), Prostanoid DP2 receptor (rGPR44), Prokineticin receptor family, Relaxin family peptide receptor family, Secretin receptor (secretin), Frizzled class receptor (Smoothened), trace amine associated receptor family, Tachykinin family, Thromboxane A2 receptor (TP), Thyrotropin-releasing hormone receptor (TRH1), and Thyroid Stimulating Hormone Receptor (TSH).

Examples of nuclear hormone receptors include, but are not limited to: Androgen receptor (AR), Estrogen related receptor alpha (ESRRA), Estrogen receptor 1 (ESR1), Nuclear receptor subfamily 1-group H-member 4 (NR1H4), Nuclear receptor subfamily 3-group C-member 1 (glucocorticoid receptor) (NR3C1), Nuclear receptor subfamily 1-group H-member 3 (Liver X receptor α) (NR1H3), Nuclear receptor subfamily 1-group H-member 2 (Liver X receptor β) (NR1H2), Nuclear receptor subfamily 1-group H-member 2 (Liver X receptor β) (NR1H2), Nuclear receptor subfamily 3-group C-member 2 (Mineralocorticoid receptor) (NR3C2), Peroxisome Proliferator Activated Receptor alpha (PPARA), Peroxisome Proliferator Activated Receptor gamma (PPARG), Peroxisome Proliferator Activated Receptor delta (PPARD), Progesterone receptor α (PGR), Progesterone receptor β (PGR), Retinoic acid receptor-alpha (RARA), Retinoic acid receptor-beta (RARB), Retinoid X receptor-alpha (RXRA), Retinoid X receptor-gamma (RXRG), Thyroid hormone receptor-alpha (THRA), Thyroid hormone receptor-beta (THRB), Retinoic acid-related orphan receptor, Liver X receptor, Farnesoid X receptor, Vitamin D receptor, Pregnane X receptor, Constitutive androstane receptor, Hepatocyte nuclear factor 4, Oestrogen receptor, Oestrogen-related receptor, Glucocortioic receptor, and Nerve growth factor-induced-B, Germ cell nuclear factor.

Examples of membrane transport proteins include, but are not limited to: ATP-binding cassette (ABC) superfamily, solute carrier (SLC) superfamily, multidrug resistance protein 1 (P-glycoprotein), organic anion transporter 1, and proteins such as EAAT3, EAAC1, EAAT1, GLUT1, GLUT2, GLUT9, GLUT10, rBAT, AE1, NBC1, KNBC, CHED2, BTR1, NABC1, CDPD, SGLT1, SGLT2, NIS, CHT1, NET, DAT, GLYT2, CRTR, BOAT1, SIT1, XT3, y+LAT1, BAT1, NHERF1, NHE6, ASBT, DMT1, DCT1, NRAMP2, NKCC2, NCC, KCC3, NACT, MCT1, MCT8, MCT12, SLD, VGLUT3, THTR1, THTR2, PIT2, GLVR2, OCTN2, URAT1, NCKX1, NCKX5, CIC, PiC, ANTI, ORNT1, AGC1, ARALAR, Citrin, STLN2, aralar2, TPC, MUP1, MCPHA, CACT, GC1, PHC, DTD, CLD, DRA, PDS, Prestin, TAT1, FATP4, ENT3, ZnT2, ZnT10, AT1, NPT2A, NPT2B, HHRH, CST, CDG2F, UGAT, UGTL, UGALT, UGT1, UGT2, FUCT1, CDG2C, NST, PAT2, G6PT1, SPX4, ZIP4, LIV4, ZIP13, LZT-Hs9, FPN1, MTP1, IREG1, RHAG, AIM1, PCFT, FLVCR1, FLVCR2, RFT1, RFT2, RFT3, OATP1B1, OATP1B3, and OATP2A1.

Examples of structural proteins include, but are not limited to: tubulin, heat shock protein, Microtubule-stabilizing proteins, Oncoprotein 18, stathmin, kinesin-8 and kinesin-14 family, Kip3, and Kif18A.

Examples of proteases include, but are not limited to ADAM (a disintegrin and metalloprotease) family.

Examples of Protein kinases include, but are not limited to: AP2 associated kinase, Homo sapiens ABL proto-oncogene 1-non-receptor tyrosine-protein kinase family, c-abl oncogene 1 receptor tyrosine kinase family, v-abl Abelson murine leukemia viral oncogene homolog 2, activin A receptor family, chaperone-ABC1 activity of bc1 complex homolog (S. pombe) (ADCK3), aarF domain containing kinase 4 (ADCK4), v-akt murine thymoma viral oncogene homolog family, anaplastic lymphoma receptor tyrosine kinase family, protein kinase A family, protein kinase B family, ankyrin repeat and kinase domain containing 1 (ANKK1), NUAK family-SNF1-like kinase, mitogen-activated protein kinase kinase kinase family aurora kinase A (AURKA), aurora kinase B (AURKB), aurora kinase C (AURKC), AXL receptor tyrosine kinase (AXL), BMP2 inducible kinase (BIKE), B lymphoid tyrosine kinase (BLK), bone morphogenetic protein receptor family, BMX non-receptor tyrosine kinase (BMX), v-raf murine sarcoma viral oncogene homolog B1 (BRAF), protein tyrosine kinase 6 (BRK), BR serine/threonine kinase family, Bruton agammaglobulinemia tyrosine kinase (BTK), calcium/calmodulin-dependent protein kinase family, cyclin-dependent kinase family, cyclin-dependent kinase-like family, CHK1 checkpoint homolog (S. pombe) (CHEK1), CHK2 checkpoint homolog (S. pombe) (CHEK2), Insulin receptor, isoform A (INSR), Insulin receptor, isoform B (INSR), rho-interacting serine/threonine kinase (CIT), v-kit Hardy-Zuckerman 4 feline sarcoma viral oncogene homolog (KIT), CDC-Like Kinase family-Hepatocyte growth factor receptor (MET), Proto-oncogene tyrosine-protein kinase receptor, colony-stimulating factor family receptor, c-src tyrosine kinase (CSK), casein kinase family, megakaryocyte-associated tyrosine kinase (CTK), death-associated protein kinase family, doublecortin-like kinase family, discoidin domain receptor tyrosine kinase, dystrophia myotonica-protein kinase (DMPK), dual-specificity tyrosine-(Y)-phosphorylation regulated kinase family, epidermal growth factor receptor family, eukaryotic translation initiation factor 2-alpha kinase 1 (EIF2AK1), EPH receptor family, Ephrin type-A receptor family, Ephrin type-B receptor family, v-erb-b2 erythroblastic leukemia viral oncogene homolog family, mitogen-activated protein kinase family, endoplasmic reticulum to nucleus signaling 1 (ERN1), PTK2 protein tyrosine kinase 2 (FAK), fer (fps/fes related) tyrosine kinase (FER). feline sarcoma oncogene (FES), Fibroblast growth factor receptor family, Gardner-Rasheed feline sarcoma viral (v-fgr) oncogene homolog (FGR), fms-related tyrosine kinase family, Fms-related tyrosine kinase family, fyn-related kinase (FRK), FYN oncogene related to SRC, cyclin G associated kinase (GAK), eukaryotic translation initiation factor 2 alpha kinase, Growth hormone receptor. G protein-coupled receptor kinase 1 (GRK1), G protein-coupled receptor kinase family, glycogen synthase kinase family, germ cell associated 2 (haspin) (HASPIN), Hemopoietic cell kinase (HCK), homeodomain interacting protein kinase family, mitogen-activated protein kinase kinase kinase kinase family, hormonally up-regulated Neu-associated kinase (HUNK), intestinal cell (MAK-like) kinase (ICK), Insulin-like growth factor 1 receptor (IGF1R), conserved helix-loop-helix ubiquitous kinase (IKK-alpha), inhibitor of kappa light polypeptide gene enhancer in B-cells-kinase beta family, insulin receptor (INSR), insulin receptor-related receptor (INSRR), interleukin-1 receptor-associated kinase family, IL2-inducible T-cell kinase (ITK), Janus kinase family, Kinase Insert Domain Receptor, v-kit Hardy-Zuckerman 4 feline sarcoma viral oncogene homolog, lymphocyte-specific protein tyrosine kinase (LCK), LIM domain kinase family, serine/threonine kinase family leucine-rich repeat kinase family, v-yes-1 Yamaguchi sarcoma viral related oncogene homolog (LYN), male germ cell-associated kinase (MAK); MAP/microtubule affinity-regulating kinase family such as microtubule associated serine/threonine kinase family, maternal embryonic leucine zipper kinase, c-mer proto-oncogene tyrosine kinase (MERTK), met proto-oncogene (hepatocyte growth factor receptor), MAP kinase interacting serine/threonine kinase family, myosin light chain kinase family, mixed lineage kinase domain-like protein isoform, CDC42 binding protein kinase family, serine/threonine kinase family, macrophage stimulating 1 receptor (c-met-related tyrosine kinase) (MST1R), mechanistic target of rapamycin (serine/threonine kinase) (MTOR), muscle-skeletal-receptor tyrosine kinase (MUSK), myosin light chain kinase family, NIMA (never in mitosis gene a)-related kinase family, serine/threonine-protein kinase NIM1 (NIM1), nemo-like kinase (NLK), oxidative-stress responsive 1 (OSR1), p21 protein (Cdc42/Rac)-activated kinase family, PAS domain containing serine/threonine kinase, Platelet-derived growth factor receptor family, 3-phosphoinositide dependent protein kinase-1 (PDPK1), Calcium-dependent protein kinase 1, phosphorylase kinase gamma family, Phosphatidylinositol 4,5-bisphosphate 3-kinase, phosphoinositide-3-kinase family, phosphatidylinositol 4-kinase family. phosphoinositide kinase, FYVE finger containing, Pim-1 oncogene (PIM1), pim-2 oncogene (PIM2), pim-3 oncogene (PIM3), phosphatidylinositol-4-phosphate 5-kinase family, phosphatidylinositol-5-phosphate 4-kinase family protein kinase, membrane associated tyrosine/threonine 1 (PKMYT1), protein kinase N family, polo-like kinase family, protein kinase C family, protein kinase D family, cGMP-dependent protein kinase family, eukaryotic translation initiation factor 2-alpha kinase 2 (PRKR), X-linked protein kinase (PRKX), Prolactin receptor (PRLR), PRP4 pre-mRNA processing factor 4 homolog B (yeast) (PRP4), PTK2B protein tyrosine kinase 2 beta (PTK2B), SIK family kinase 3 (QSK), v-raf-1 murine leukemia viral oncogene homolog 1 (RAF1), Neurotrophic tyrosine kinase receptor type family, receptor (TNFRSF)-interacting serine-threonine kinase family, dual serine/threonine and tyrosine protein kinase (RIPK5), Rho-associated, coiled-coil containing protein kinase family, c-ros oncogene 1, receptor tyrosine kinase (ROS1), ribosomal protein S6 kinase family, SH3-binding domain kinase 1 (SBK1), serum/glucocorticoid regulated kinase family, Putative uncharacterized serine/threonine-protein kinase (Sugen kinase 110) (SgK110), salt-inducible kinase family, SNF related kinase (SNRK), src-related kinase, SFRS protein kinase family; Spleen tyrosine kinase (SYK) such as TAO kinase family; TANK-binding kinase 1 (TBK1) such as tec protein tyrosine kinase (TEC), testis-specific kinase 1 (TESK1), transforming growth factor, beta receptor family, tyrosine kinase with immunoglobulin-like and EGF-like domains 1 (TIE1), TEK tyrosine kinase, endothelial (TIE2), Angiopoietin-1 receptor (Tie2), tousled-like kinase family, TRAF2 and NCK interacting kinase (TN IK), non-receptor tyrosine kinase family, TNNI3 interacting kinase (TNNI3K), transient receptor potential cation channel, testis-specific serine kinase family, TTK protein kinase (TTK), TXK tyrosine kinase (TXK), Tyrosine kinase 2 (TYK2), TYRO3 protein tyrosine kinase (TYRO3), unc-51-like kinase family, phosphatidylinositol 3-kinase, vaccinia related kinase 2 (VRK2), WEE1 homolog family, WNK lysine deficient protein kinase family, v-yes-1 Yamaguchi sarcoma viral oncogene homolog 1 (YES), sterile alpha motif and leucine zipper containing kinase AZK (ZAK), and zeta-chain (TCR) associated protein kinase 70 kDa (ZAP70).

Cell therapy using cells that are derived primarily from: endoderm such as Exocrine secretory epithelial cells and Hormone-secreting cells; ectoderm such as Keratinizing epithelial cells, Wet stratified barrier epithelial cells, Sensory transducer cells, Autonomic neuron cells, Sense organ and peripheral neuron supporting cells, Central nervous system neurons and glial cells, Lens cells; mesoderm such as Metabolism and storage cells, Barrier function cells (lung, gut, exocrine glands and urogenital tract), Extracellular matrix cells, Contractile cells, Blood and immune system cells, Germ cells, Nurse cell, Interstitial cells and combinations thereof. Additionally, in the scope of the invention are cells that are genetically, chemically or physically altered or otherwise modified.

Examples of Exocrine secretory epithelial cells include but are not limited to: Salivary gland mucous cell, Salivary gland number 1, Von Ebner's gland cell in tongue, Mammary gland cell, Lacrimal gland cell, Ceruminous gland cell in ear, Eccrine sweat gland dark cell, Eccrine sweat gland clear cell, Apocrine sweat gland cell, Gland of Moll cell in eyelid, Sebaceous gland cell, Bowman's gland cell in nose, Brunner's gland cell in duodenum, Seminal vesicle cell, Prostate gland cell, Bulbourethral gland cell, Bartholin's gland cell, Gland of Littre cell, Uterus endometrium cell, Isolated goblet cell of respiratory and digestive tracts, Stomach lining mucous cell, Gastric gland zymogenic cell, Gastric gland oxyntic cell, Pancreatic acinar cell, Paneth cell of small intestine, Type II pneumocyte of lung, and Clara cell of lung; Hormone-secreting cells including, but not limited to: Anterior pituitary cells, Intermediate pituitary cell, Magnocellular neurosecretory cells, Gut and respiratory tract cells, Thyroid gland cells, Parathyroid gland cells, Adrenal gland cells, Leydig cell of testes secreting testosterone, Theca interna cell of ovarian follicle secreting estrogen, Corpus luteum cell of ruptured ovarian follicle secreting progesterone, Juxtaglomerular cell, Macula densa cell of kidney, Peripolar cell of kidney, Mesangial cell of kidney, and Pancreatic islets; Keratinizing epithelial cells including, but not limited to: Epidermal keratinocyte, Epidermal basal cell, Keratinocyte of fingernails and toenails, Nail bed basal cell, Medullary hair shaft cell, Cortical hair shaft cell, Cuticular hair shaft cell, Cuticular hair root sheath cell, Hair root sheath cell of Huxley's layer, Hair root sheath cell of Henle's layer, External hair root sheath cell, and Hair matrix cell; Wet stratified barrier epithelial cells including, but not limited to: Surface epithelial cell of stratified squamous epithelium and basal cell of epithelia of cornea, tongue, oral cavity, esophagus, anal canal, distal urethra and vagina, and Urinary epithelium cell; Sensory transducer cells including, but not limited to: Auditory inner hair cell of organ of Corti, Auditory outer hair cell of organ of Corti, Basal cell of olfactory epithelium, Cold-sensitive primary sensory neurons, Heat-sensitive primary sensory neurons, Merkel cell of epidermis, Olfactory receptor neuron, Pain-sensitive primary sensory neurons, Photoreceptor cells of retina in eye, Proprioceptive primary sensory neurons, Touch-sensitive primary sensory neurons, Type I carotid body cell, Type II carotid body cell, Type I hair cell of vestibular system of ear, Type II hair cell of vestibular system of ear, and Type I taste bud cell; Autonomic neuron cells including, but not limited to: Cholinergic neural cell, Adrenergic neural cell, and Peptidergic neural cell; Sense organ and peripheral neuron supporting cells including, but not limited to: Inner pillar cell of organ of Corti, Outer pillar cell of organ of Corti, Inner phalangeal cell of organ of Corti, Outer phalangeal cell of organ of Corti, Border cell of organ of Corti, Hensen cell of organ of Corti, Vestibular apparatus supporting cell, Taste bud supporting cell, Olfactory epithelium supporting cell, Schwann cell, Satellite glial cell, and Enteric glial cell; Central nervous system neurons and glial cells including, but not limited to: Astrocyte, Neuron cells, Oligodendrocyte, and Spindle neuron; Lens cells including, but not limited to: Anterior lens epithelial cell, and Crystallin-containing lens fiber cell; Metabolism and storage cells including, but not limited to: Adipocytes, and Liver lipocyte; Barrier function cells including, but not limited to: Kidney parietal cell, Kidney glomerulus podocyte, Kidney proximal tubule brush border cell, Loop of Henle thin segment cell, Kidney distal tubule cell, Kidney collecting duct cell, Principal cells, Intercalated cells, Type I pneumocyte, Pancreatic duct cell, Nonstriated duct cell, Principal cell, Intercalated cell, Duct cell, Intestinal brush border cell, Exocrine gland striated duct cell, Gall bladder epithelial cell, Ductulus efferens nonciliated cell, Epididymal principal cell, and Epididymal basal cell; Extracellular matrix cells including, but not limited to: Ameloblast epithelial cell, Planum semilunatum epithelial cell of vestibular system of ear, Organ of Corti interdental epithelial cell, Loose connective tissue fibroblasts, Corneal fibroblasts, Tendon fibroblasts, Bone marrow reticular tissue fibroblasts, Other nonepithelial fibroblasts, Pericyte, Nucleus pulposus cell of intervertebral disc, Cementoblast/cementocyte, Odontoblast/odontocyte, Hyaline cartilage chondrocyte, Fibrocartilage chondrocyte, Elastic cartilage chondrocyte, Osteoblast/osteocyte, Osteoprogenitor cell, Hyalocyte of vitreous body of eye, Stellate cell of perilymphatic space of ear, Hepatic stellate cell, and Pancreatic stelle cell; Contractile cells including, but not limited to: Skeletal muscle cell, Satellite cell, Heart muscle cells, Smooth muscle cell, Myoepithelial cell of iris, and Myoepithelial cell of exocrine glands; Blood and immune system cells including, but not limited to: Erythrocyte, Megakaryocyte, Monocyte, Connective tissue macrophage, Epidermal Langerhans cell, Osteoclast, Dendritic cell, Microglial cell, Neutrophil granulocyte, Eosinophil granulocyte, Basophil granulocyte, Hybridoma cell, Mast cell, Helper T cell, Suppressor T cell, Cytotoxic T cell, Natural Killer T cell, B cell, Natural killer cell, Reticulocyte, Stem cells, and committed progenitors for the blood and immune system; Germ cells including, but not limited to: Oogonium/Oocyte, Spermatid, Spermatocyte, Spermatogonium cell, and Spermatozoon; Nurse cell including, but not limited to: Ovarian follicle cell, and Sertoli cell, Thymus epithelial cell; Interstitial cells including, but not limited to: Interstitial kidney cells and any combination of the foregoing.

Non-limiting examples of other known biologics include, but are not limited to: Abbosynagis, Abegrin, Actemra, AFP-Cide, Antova, Arzerra, Aurexis, Avastin, Benlysta, Bexxar, Blontress, Bosatria, Campath, CEA-Cide, CEA-Scan, Cimzia, Cyramza, Ektomab, Erbitux, FibriScint, Gazyva, Herceptin, hPAM4-Cide, HumaSPECT, HuMax-CD4, HuMax-EGFr, Humira, HuZAF, Hybri-ceaker, Ilaris, Indimacis-125, Kadcyla, Lemtrada, LeukArrest, LeukoScan, Lucentis, Lymphomun, LymphoScan, LymphoStat-B, MabThera, Mycograb, Mylotarg, Myoscint, NeutroSpec, Numax, Nuvion, Omnitarg, Opdivo, Orthoclone OKT3, OvaRex, Panorex, Prolia, Prostascint, Raptiva, Remicade, Removab, Rencarex, ReoPro, Rexomun, Rituxan, RoActemra, Scintimun, Simponi, Simulect, Soliris, Stelara, Synagis, Tactress, Theracim, Theragyn, Theraloc, Tysabri, Vectibix, Verluma, Xolair, Yervoy, Zenapax, and Zevalin and combinations thereof.

Non-limiting examples of known Monoclonal antibodies include, but are not limited to: 3F8, 8H9, Abagovomab, Abciximab, Abituzumab, Abrilumab, Actoxumab, Adalimumab, Adecatumumab, Aducanumab, Afasevikumab, Afelimomab, Afutuzumab, Alacizumab pegol, ALD518, ALD403, Alemtuzumab, Alirocumab, Altumomab pentetate, Amatuximab, AMG 334, Anatumomab mafenatox, Anetumab ravtansine, Anifrolumab, Anrukinzumab, Apolizumab, Arcitumomab, Ascrinvacumab, Aselizumab, Atezolizumab, Atinumab, Atlizumab, Atorolimumab, Avelumab, Bapineuzumab, Basiliximab, Bavituximab, Bectumomab, Begelomab, Belimumab, Benralizumab, Bertilimumab, Besilesomab, Bevacizumab, Bezlotoxumab, Biciromab, Bimagrumab, Bimekizumab, Bivatuzumab mertansine, Bleselumab, Blinatumomab, Blontuvetmab, Blosozumab, Bococizumab, Brazikumab, Brentuximab vedotin, Briakinumab, Brodalumab, Brolucizumab, Brontictuzumab, Burosumab, Cabiralizumab, Canakinumab, Cantuzumab mertansine, Cantuzumab ravtansine, Caplacizumab, Capromab pendetide, Carlumab, Carotuximab, Catumaxomab, cBR96-doxorubicin immunoconjugate, Cedelizumab, Cergutuzumab amunaleukin, Certolizumab pegol, Cetuximab, Citatuzumab bogatox, Cixutumumab, Clazakizumab, Clenoliximab, Clivatuzumab tetraxetan, Codrituzumab, Coltuximab ravtansine, Conatumumab, Concizumab, CR6261, Crenezumab, Crotedumab, Dacetuzumab, Daclizumab, Dalotuzumab, Dapirolizumab pegol, Daratumumab, Dectrekumab, Demcizumab, Denintuzumab mafodotin, Denosumab, Depatuxizumab mafodotin, Derlotuximab biotin, Detumomab, Dinutuximab, Diridavumab, Domagrozumab, Dorlimomab aritox, Drozitumab, Duligotumab, Dupilumab, Durvalumab, Dusigitumab, Ecromeximab, Eculizumab, Edobacomab, Edrecolomab, Efalizumab, Efungumab, Eldelumab, Elgemtumab, Elotuzumab, Elsilimomab, Emactuzumab, Emibetuzumab, Emicizumab, Enavatuzumab, Enfortumab vedotin, Enlimomab pegol, Enoblituzumab, Enokizumab, Enoticumab, Ensituximab, Epitumomab cituxetan, Epratuzumab, Erenumab, Erlizumab, Ertumaxomab, Etaracizumab, Etrolizumab, Evinacumab, Evolocumab, Exbivirumab, Fanolesomab, Faralimomab, Farletuzumab, Fasinumab, FBTA05, Felvizumab, Fezakinumab, Fibatuzumab, Ficlatuzumab, Figitumumab, Firivumab, Flanvotumab, Fletikumab, Fontolizumab, Foralumab, Foravirumab, Fresolimumab, Fulranumab, Futuximab, Galcanezumab, Galiximab, Ganitumab, Gantenerumab, Gavilimomab, Gemtuzumab ozogamicin, Gevokizumab, Girentuximab, Glembatumumab vedotin, Golimumab, Gomiliximab, Guselkumab, Ibalizumab, Ibritumomab tiuxetan, Icrucumab, Idarucizumab, Igovomab, IMA-638, IMAB362, Imalumab, Imciromab, Imgatuzumab, Inclacumab, Indatuximab ravtansine, Indusatumab vedotin, Inebilizumab, Infliximab, Inolimomab, Inotuzumab ozogamicin, Intetumumab, Ipilimumab, Iratumumab, Isatuximab, Itolizumab, Ixekizumab, Keliximab, Labetuzumab, Lambrolizumab, Lampalizumab, Lanadelumab, Landogrozumab, Laprituximab emtansine, LBR-101/PF0442g7429, Lebrikizumab, Lemalesomab, Lendalizumab, Lenzilumab, Lerdelimumab, Lexatumumab, Libivirumab, Lifastuzumab vedotin, Ligelizumab, Lilotomab satetraxetan, Lintuzumab, Lirilumab, Lodelcizumab, Lokivetmab, Lorvotuzumab mertansine, Lucatumumab, Lulizumab pegol, Lumiliximab, Lumretuzumab, LY2951742, Mapatumumab, Margetuximab, Maslimomab, Matuzumab, Mavrilimumab, Mepolizumab, Metelimumab, Milatuzumab, Minretumomab, Mirvetuximab soravtansine, Mitumomab, Mogamulizumab, Monalizumab, Morolimumab, Motavizumab, Moxetumomab pasudotox, Muromonab-CD3, Nacolomab tafenatox, Nam ilumab, Naptumomab estafenatox, Naratuximab emtansine, Narnatumab, Natalizumab, Navicixizumab, Navivumab, Nebacumab, Necitumumab, Nemolizumab, Nerelimomab, Nesvacumab, Nimotuzumab, Nivolumab, Nofetumomab merpentan, Obiltoxaximab, Obinutuzumab, Ocaratuzumab, Ocrelizumab, Odulimomab, Ofatumumab, Olaratumab, Olokizumab, Omalizumab, Onartuzumab, Ontuxizumab, Opicinumab, Oportuzumab monatox, Oregovomab, Orticumab, Otelixizumab, Otlertuzumab, Oxelumab, Ozanezumab, Ozoralizumab, Pagibaximab, Palivizumab, Pamrevlumab, Panitumumab, Pankomab, Panobacumab, Parsatuzumab, Pascolizumab, Pasotuxizumab, Pateclizumab, Patritumab, Pembrolizumab, Pemtumomab, Perakizumab, Pertuzumab, Pexelizumab, Pidilizumab, Pinatuzumab vedotin, Pintumomab, Placulumab, Plozalizumab, Pogalizumab, Polatuzumab vedotin, Ponezumab, Prezalizumab, Priliximab, Pritoxaximab, Pritumumab, PRO 140, Quilizumab, Racotumomab, Radretumab, Rafivirumab, Ralpancizumab, Ramucirumab, Ranibizumab, Raxibacumab, Refanezumab, Regavirumab, Reslizumab, Rilotumumab, Rinucumab, Risankizumab, Rituximab, Rivabazumab pegol, Robatumumab, Roledumab, Romosozumab, Rontalizumab, Rovalpituzumab tesirine, Rovelizumab, Ruplizumab, Sacituzumab govitecan, Samalizumab, Sapelizumab, Sarilumab, Satumomab pendetide, Secukinumab, Seribantumab, Setoxaximab, Sevirumab, SGN-CD19A, SGN-CD33A, Sibrotuzumab, Sifalimumab, Siltuximab, Simtuzumab, Siplizumab, Sirukumab, Sofituzumab vedotin, Solanezumab, Solitomab, Sonepcizumab, Sontuzumab, Stamulumab, Sulesomab, Suvizumab, Tabalumab, Tacatuzumab tetraxetan, Tadocizumab, Talizumab, Tamtuvetmab, Tanezumab, Taplitumomab paptox, Tarextumab, Tefibazumab, Telimomab aritox, Tenatumomab, Teneliximab, Teplizumab, Teprotumumab, Tesidolumab, Tetulomab, Tezepelumab, TGN1412, Ticilimumab, Tigatuzumab, Tildrakizumab, Timolumab, Tisotumab vedotin, TNX-650, Tocilizumab, Toralizumab, Tosatoxumab, Tositumomab, Tovetumab, Tralokinumab, Trastuzumab, Trastuzumab emtansine, TRBS07, Tregalizumab, Tremelimumab, Trevogrumab, Tucotuzumab celmoleukin, Tuvirumab, Ublituximab, Ulocuplumab, Urelumab, Urtoxazumab, Ustekinumab, Utomilumab, Vadastuximab talirine, Vandortuzumab vedotin, Vantictumab, Vanucizumab, Vapaliximab, Varlilumab, Vatelizumab, Vedolizumab, Veltuzumab, Vepalimomab, Vesencumab, Visilizumab, Vobarilizumab, Volociximab, Vorsetuzumab mafodotin, Votumumab, Xentuzumab, Zalutumumab, Zanolimumab, Zatuximab, Ziralimumab, and Zolimomab aritox and combinations thereof.

Examples of vaccines developed for viral diseases include, but are not limited to: Hepatitis A vaccine, Hepatitis B vaccine, Hepatitis E vaccine, HPV vaccine, Influenza vaccine, Japanese encephalitis vaccine, MMR vaccine, MMRV vaccine, Polio vaccine, Rabies vaccine, Rotavirus vaccine, Varicella vaccine, Shingles vaccine, Smallpox vaccine, Yellow Fever vaccine, Adenovirus vaccine, Coxsackie B virus vaccine, Cytomegalovirus vaccine, Dengue vaccine for humans, Eastern Equine encephalitis virus vaccine for humans, Ebola vaccine, Enterovirus 71 vaccine, Epstein-Barr vaccine, Hepatitis C vaccine, HIV vaccine, HTLV-1 T-lymphotropic leukemia vaccine for humans, Marburg virus disease vaccine, Norovirus vaccine, Respiratory syncytial virus vaccine for humans, Severe acute respiratory syndrome (SARS) vaccine, West Nile virus vaccine for humans; Examples of bacterial diseases include but are not limited to: Anthrax vaccines, DPT vaccine, Q fever vaccine, Hib vaccine, Tuberculosis (BCG) vaccine, Meningococcal vaccine, Typhoid vaccine, Pneumococcal conjugate vaccine, Pneumococcal polysaccharide vaccine, Cholera vaccine, Caries vaccine, Ehrlichiosis vaccine, Leprosy vaccine, Lyme disease vaccine, Staphylococcus aureus vaccine, Streptococcus pyogenes vaccine, Syphilis vaccine, Tularemia vaccine, and Yersinia pestis vaccine; Examples of parasitic diseases include, but are not limited to: Malaria vaccine, Schistosomiasis vaccine, Chagas disease vaccine, Hookworm vaccine, Onchocerciasis river blindness vaccine for humans, Trypanosomiasis vaccine, and Visceral leishmaniasis vaccine; Examples of non-infectious diseases include, but are not limited to: Alzheimer's disease amyloid protein vaccine, Breast cancer vaccine, Ovarian cancer vaccine, Prostate cancer vaccine, and Talimogene laherparepvec (T-VEC); also vaccines including, but not limited to the following trade names: ACAM2000, ActHIB, Adacel, Afluria, AFLURIA QUADRIVALENT, Agriflu, BCG Vaccine, BEXSERO, Biothrax, Boostrix, Cervarix, Comvax, DAPTACEL, DECAVAC, Engerix-B, FLUAD, Fluarix, Fluarix Quadrivalent, Flublok, Flucelvax, Flucelvax Quadrivalent, FluLaval, FluMist, FluMist Quadrivalent, Fluvirin, Fluzone Quadrivalent, Fluzone, Fluzone High-Dose and Fluzone Intradermal, Gardasil, Gardasil 9, Havrix, Hiberix, Imovax, Infanrix, IPOL, Ixiaro, JE-Vax, KINRIX, Menactra, MenHibrix, Menomune-A/C/Y/W-135, Menveo, M-M-R II, M-M-Vax, Pediarix, PedvaxHIB, Pentacel, Pneumovax 23, Poliovax, Prevnar, Prevnar 13, ProQuad, Quadracel, Quadrivalent, RabAvert, Recombivax HB, ROTARIX, RotaTeq, TENIVAC, TICE BCG, Tripedia, TRUMENBA, Twinrix, TYPHIM Vi, VAQTA, Varivax, Vaxchora, Vivotif, YF-Vax, Zostavax, and combinations thereof.

Examples of injectable drugs include, but are not limited to: Ablavar (Gadofosveset Trisodium Injection), Abarelix Depot, Abobotulinumtoxin A Injection (Dysport), ABT-263, ABT-869, ABX-EFG, Accretropin (Somatropin Injection), Acetadote (Acetylcysteine Injection), Acetazolamide Injection (Acetazolamide Injection), Acetylcysteine Injection (Acetadote), Actemra (Tocilizumab Injection), Acthrel (Corticorelin Ovine Triflutate for Injection), Actummune, Activase, Acyclovir for Injection (Zovirax Injection), Adacel, Adalimumab, Adenoscan (Adenosine Injection), Adenosine Injection (Adenoscan), Adrenaclick, AdreView (Iobenguane 1123 Injection for Intravenous Use), Afluria, Ak-Fluor (Fluorescein Injection), Aldurazyme (Laronidase), Alglucerase Injection (Ceredase), Alkeran Injection (Melphalan Hcl Injection), Allopurinol Sodium for Injection (Aloprim), Aloprim (Allopurinol Sodium for Injection), Alprostadil, Alsuma (Sumatriptan Injection), ALTU-238, Amino Acid Injections, Aminosyn, Apidra, Apremilast, Alprostadil Dual Chamber System for Injection (Caverject Impulse), AMG 009, AMG 076, AMG 102, AMG 108, AMG 114, AMG 162, AMG 220, AMG 221, AMG 222, AMG 223, AMG 317, AMG 379, AMG 386, AMG 403, AMG 477, AMG 479, AMG 517, AMG 531, AMG 557, AMG 623, AMG 655, AMG 706, AMG 714, AMG 745, AMG 785, AMG 811, AMG 827, AMG 837, AMG 853, AMG 951, Amiodarone HCl Injection (Amiodarone HCl Injection), Amobarbital Sodium Injection (Amytal Sodium), Amytal Sodium (Amobarbital Sodium Injection), Anakinra, Anti-Abeta, Anti-Beta7, Anti-Beta20, Anti-CD4, Anti-CD20, Anti-CD40, Anti-IFNalpha, Anti-IL13, Anti-OX40L, Anti-oxLDS, Anti-NGF, Anti-NRP1, Arixtra, Amphadase (Hyaluronidase Inj), Ammonul (Sodium Phenylacetate and Sodium Benzoate Injection), Anaprox, Anzemet Injection (Dolasetron Mesylate Injection), Apidra (Insulin Glulisine [rDNA origin] Inj), Apomab, Aranesp (darbepoetin alfa), Argatroban (Argatroban Injection), Arginine Hydrochloride Injection (R-Gene 10, Aristocort, Aristospan, Arsenic Trioxide Injection (Trisenox), Articane HCl and Epinephrine Injection (Septocaine), Arzerra (Ofatumumab Injection), Asclera (Polidocanol Injection), Ataluren, Ataluren-DMD, Atenolol Inj (Tenormin I.V. Injection), Atracurium Besylate Injection (Atracurium Besylate Injection), Avastin, Azactam Injection (Aztreonam Injection), Azithromycin (Zithromax Injection), Aztreonam Injection (Azactam Injection), Baclofen Injection (Lioresal Intrathecal), Bacteriostatic Water (Bacteriostatic Water for Injection), Baclofen Injection (Lioresal Intrathecal), Bal in Oil Ampules (Dimercarprol Injection), BayHepB, BayTet, Benadryl, Bendamustine Hydrochloride Injection (Treanda), Benztropine Mesylate Injection (Cogentin), Betamethasone Injectable Suspension (Celestone Soluspan), Bexxar, Bicillin C-R 900/300 (Penicillin G Benzathine and Penicillin G Procaine Injection), Blenoxane (Bleomycin Sulfate Injection), Bleomycin Sulfate Injection (Blenoxane), Boniva Injection (Ibandronate Sodium Injection), Botox Cosmetic (OnabotulinumtoxinA for Injection), BR3-FC, Bravelle (Urofollitropin Injection), Bretylium (Bretylium Tosylate Injection), Brevital Sodium (Methohexital Sodium for Injection), Brethine, Briobacept, BTT-1023, Bupivacaine HCI, Byetta, Ca-DTPA (Pentetate Calcium Trisodium Inj), Cabazitaxel Injection (Jevtana), Caffeine Alkaloid (Caffeine and Sodium Benzoate Injection), Calcijex Injection (Calcitrol), Calcitrol (Calcijex Injection), Calcium Chloride (Calcium Chloride Injection 10%), Calcium Disodium Versenate (Edetate Calcium Disodium Injection), Campath (Altemtuzumab), Camptosar Injection (Irinotecan Hydrochloride), Canakinumab Injection (Ilaris), Capastat Sulfate (Capreomycin for Injection), Capreomycin for Injection (Capastat Sulfate), Cardiolite (Prep kit for Technetium Tc99 Sestamibi for Injection), Carticel, Cathflo, Cefazolin and Dextrose for Injection (Cefazolin Injection), Cefepime Hydrochloride, Cefotaxime, Ceftriaxone, Cerezyme, Carnitor Injection, Caverject, Celestone Soluspan, Celsior, Cerebyx (Fosphenytoin Sodium Injection), Ceredase (Alglucerase Injection), Ceretec (Technetium Tc99m Exametazime Injection), Certolizumab, CF-101, Chloramphenicol Sodium Succinate (Chloramphenicol Sodium Succinate Injection), Chloramphenicol Sodium Succinate Injection (Chloramphenicol Sodium Succinate), Cholestagel (Colesevelam HCL), Choriogonadotropin Alfa Injection (Ovidrel), Cimzia, Cisplatin (Cisplatin Injection), Clolar (Clofarabine Injection), Clomiphine Citrate, Clonidine Injection (Duraclon), Cogentin (Benztropine Mesylate Injection), Colistimethate Injection (Coly-Mycin M), Coly-Mycin M (Colistimethate Injection), Compath, Conivaptan Hcl Injection (Vaprisol), Conjugated Estrogens for Injection (Premarin Injection), Copaxone, Corticorelin Ovine Triflutate for Injection (Acthrel), Corvert (Ibutilide Fumarate Injection), Cubicin (Daptomycin Injection), CF-101, Cyanokit (Hydroxocobalamin for Injection), Cytarabine Liposome Injection (DepoCyt), Cyanocobalamin, Cytovene (ganciclovir), D.H.E. 45, Dacetuzumab, Dacogen (Decitabine Injection), Dalteparin, Dantrium IV (Dantrolene Sodium for Injection), Dantrolene Sodium for Injection (Dantrium IV), Daptomycin Injection (Cubicin), Darbepoietin Alfa, DDAVP Injection (Desmopressin Acetate Injection), Decavax, Decitabine Injection (Dacogen), Dehydrated Alcohol (Dehydrated Alcohol Injection), Denosumab Injection (Prolia), Delatestryl, Delestrogen, Delteparin Sodium, Depacon (Valproate Sodium Injection), Depo Medrol (Methylprednisolone Acetate Injectable Suspension), DepoCyt (Cytarabine Liposome Injection), DepoDur (Morphine Sulfate XR Liposome Injection), Desmopressin Acetate Injection (DDAVP Injection), Depo-Estradiol, Depo-Provera 104 mg/ml, Depo-Provera 150 mg/ml, Depo-Testosterone, Dexrazoxane for Injection, Intravenous Infusion Only (Totect), Dextrose/Electrolytes, Dextrose and Sodium Chloride Inj (Dextrose 5% in 0.9% Sodium Chloride), Dextrose, Diazepam Injection (Diazepam Injection), Digoxin Injection (Lanoxin Injection), Dilaudid-HP (Hydromorphone Hydrochloride Injection), Dimercarprol Injection (Bal in Oil Ampules), Diphenhydramine Injection (Benadryl Injection), Dipyridamole Injection (Dipyridamole Injection), DMOAD, Docetaxel for Injection (Taxotere), Dolasetron Mesylate Injection (Anzemet Injection), Doribax (Doripenem for Injection), Doripenem for Injection (Doribax), Doxercalciferol Injection (Hectorol Injection), Doxil (Doxorubicin Hcl Liposome Injection), Doxorubicin Hcl Liposome Injection (Doxil), Duraclon (Clonidine Injection), Duramorph (Morphine Injection), Dysport (Abobotulinumtoxin A Injection), Ecallantide Injection (Kalbitor), EC-Naprosyn (naproxen), Edetate Calcium Disodium Injection (Calcium Disodium Versenate), Edex (Alprostadil for Injection), Engerix, Edrophonium Injection (Enlon), Eliglustat Tartate, Eloxatin (Oxaliplatin Injection), Emend Injection (Fosaprepitant Dimeglumine Injection), Enalaprilat Injection (Enalaprilat Injection), Enlon (Edrophonium Injection), Enoxaparin Sodium Injection (Lovenox), Eovist (Gadoxetate Disodium Injection), Enbrel (etanercept), Enoxaparin, Epicel, Epinepherine, Epipen, Epipen Jr., Epratuzumab, Erbitux, Ertapenem Injection (Invanz), Erythropoieten, Essential Amino Acid Injection (Nephramine), Estradiol Cypionate, Estradiol Valerate, Etanercept, Exenatide Injection (Byetta), Evlotra, Fabrazyme (Adalsidase beta), Famotidine Injection, FDG (Fludeoxyglucose F 18 Injection), Feraheme (Ferumoxytol Injection), Feridex I.V. (Ferumoxides Injectable Solution), Fertinex, Ferumoxides Injectable Solution (Feridex I.V.), Ferumoxytol Injection (Feraheme), Flagyl Injection (Metronidazole Injection), Fluarix, Fludara (Fludarabine Phosphate), Fludeoxyglucose F 18 Injection (FDG), Fluorescein Injection (Ak-Fluor), Follistim AQ Cartridge (Follitropin Beta Injection), Follitropin Alfa Injection (Gonal-f RFF), Follitropin Beta Injection (Follistim AQ Cartridge), Folotyn (Pralatrexate Solution for Intravenous Injection), Fondaparinux, Forteo (Teriparatide (rDNA origin) Injection), Fostamatinib, Fosaprepitant Dimeglumine Injection (Emend Injection), Foscarnet Sodium Injection (Foscavir), Foscavir (Foscarnet Sodium Injection), Fosphenytoin Sodium Injection (Cerebyx), Fospropofol Disodium Injection (Lusedra), Fragmin, Fuzeon (enfuvirtide), GA101, Gadobenate Dimeglumine Injection (Multihance), Gadofosveset Trisodium Injection (Ablavar), Gadoteridol Injection Solution (ProHance), Gadoversetamide Injection (OptiMARK), Gadoxetate Disodium Injection (Eovist), Ganirelix (Ganirelix Acetate Injection), Gardasil, GC1008, GDFD, Gemtuzumab Ozogamicin for Injection (Mylotarg), Genotropin, Gentamicin Injection, GENZ-112638, Golimumab Injection (Simponi Injection), Gonal-f RFF (Follitropin Alfa Injection), Granisetron Hydrochloride (Kytril Injection), Gentamicin Sulfate, Glatiramer Acetate, Glucagen, Glucagon, HAE1, Haldol (Haloperidol Injection), Havrix, Hectorol Injection (Doxercalciferol Injection), Hedgehog Pathway Inhibitor, Heparin, Herceptin, hG-CSF, Humalog, Human Growth Hormone, Humatrope, HuMax, Humegon, Humira, Humulin, Ibandronate Sodium Injection (Boniva Injection), Ibuprofen Lysine Injection (NeoProfen), Ibutilide Fumarate Injection (Corvert), Idamycin PFS (Idarubicin Hydrochloride Injection), Idarubicin Hydrochloride Injection (Idamycin PFS), Ilaris (Canakinumab Injection), Imipenem and Cilastatin for Injection (Primaxin I.V.), Imitrex, Incobotulinumtoxin A for Injection (Xeomin), Increlex (Mecasermin [rDNA origin] Injection), Indocin IV (Indomethacin Inj), Indomethacin Inj (Indocin IV), Infanrix, Innohep, Insulin, Insulin Aspart [rDNA origin] Inj (NovoLog), Insulin Glargine [rDNA origin] Injection (Lantus), Insulin Glulisine [rDNA origin] Inj (Apidra), Interferon alfa-2b, Recombinant for Injection (Intron A), Intron A (Interferon alfa-2b, Recombinant for Injection), Invanz (Ertapenem Injection), Invega Sustenna (Paliperidone Palmitate Extended-Release Injectable Suspension), Invirase (saquinavir mesylate), lobenguane 1123 Injection for Intravenous Use (AdreView), lopromide Injection (Ultravist), loversol Injection (Optiray Injection), Iplex (Mecasermin Rinfabate [rDNA origin] Injection), Iprivask, Irinotecan Hydrochloride (Camptosar Injection), Iron Sucrose Injection (Venofer), Istodax (Romidepsin for Injection), Itraconazole Injection (Sporanox Injection), Jevtana (Cabazitaxel Injection), Jonexa, Kalbitor (Ecallantide Injection), KCL in D5NS (Potassium Chloride in 5% Dextrose and Sodium Chloride Injection), KCL in D5W, KCL in NS, Kenalog 10 Injection (Triamcinolone Acetonide Injectable Suspension), Kepivance (Palifermin), Keppra Injection (Levetiracetam), Keratinocyte, KFG, Kinase Inhibitor, Kineret (Anakinra), Kinlytic (Urokinase Injection), Kinrix, Klonopin (clonazepam), Kytril Injection (Granisetron Hydrochloride), lacosamide Tablet and Injection (Vimpat), Lactated Ringer's, Lanoxin Injection (Digoxin Injection), Lansoprazole for Injection (Prevacid I.V.), Lantus, Leucovorin Calcium (Leucovorin Calcium Injection), Lente (L), Leptin, Levemir, Leukine Sargramostim, Leuprolide Acetate, Levothyroxine, Levetiracetam (Keppra Injection), Lovenox, Levocarnitine Injection (Carnitor Injection), Lexiscan (Regadenoson Injection), Lioresal Intrathecal (Baclofen Injection), Liraglutide [rDNA] Injection (Victoza), Lovenox (Enoxaparin Sodium Injection), Lucentis (Ranibizumab Injection), Lumizyme, Lupron (Leuprolide Acetate Injection), Lusedra (Fospropofol Disodium Injection), Maci, Magnesium Sulfate (Magnesium Sulfate Injection), Mannitol Injection (Mannitol IV), Marcaine (Bupivacaine Hydrochloride and Epinephrine Injection), Maxipime (Cefepime Hydrochloride for Injection), MDP Multidose Kit of Technetium Injection (Technetium Tc99m Medronate Injection), Mecasermin [rDNA origin] Injection (Increlex), Mecasermin Rinfabate [rDNA origin] Injection (Iplex), Melphalan Hcl Injection (Alkeran Injection), Methotrexate, Menactra, Menopur (Menotropins Injection), Menotropins for Injection (Repronex), Methohexital Sodium for Injection (Brevital Sodium), Methyldopate Hydrochloride Injection, Solution (Methyldopate Hcl), Methylene Blue (Methylene Blue Injection), Methylprednisolone Acetate Injectable Suspension (Depo Medrol), MetMab, Metoclopramide Injection (Reglan Injection), Metrodin (Urofollitropin for Injection), Metronidazole Injection (Flagyl Injection), Miacalcin, Midazolam (Midazolam Injection), Mimpara (Cinacalet), Minocin Injection (Minocycline Inj), Minocycline Inj (Minocin Injection), Mipomersen, Mitoxantrone for Injection Concentrate (Novantrone), Morphine Injection (Duramorph), Morphine Sulfate XR Liposome Injection (DepoDur), Morrhuate Sodium (Morrhuate Sodium Injection), Motesanib, Mozobil (Plerixafor Injection), Multihance (Gadobenate Dimeglumine Injection), Multiple Electrolytes and Dextrose Injection, Multiple Electrolytes Injection, Mylotarg (Gemtuzumab Ozogamicin for Injection), Myozyme (Alglucosidase alfa), Nafcillin Injection (Nafcillin Sodium), Nafcillin Sodium (Nafcillin Injection), Naltrexone XR Inj (Vivitrol), Naprosyn (naproxen), NeoProfen (Ibuprofen Lysine Injection), Nandrol Decanoate, Neostigmine Methylsulfate (Neostigmine Methylsulfate Injection), NEO-GAA, NeoTect (Technetium Tc 99m Depreotide Injection), Nephramine (Essential Amino Acid Injection), Neulasta (pegfilgrastim), Neupogen (Filgrastim), Novolin, Novolog, NeoRecormon, Neutrexin (Trimetrexate Glucuronate Inj), NPH (N), Nexterone (Amiodarone HCl Injection), Norditropin (Somatropin Injection), Normal Saline (Sodium Chloride Injection), Novantrone (Mitoxantrone for Injection Concentrate), Novolin 70/30 Innolet (70% NPH, Human Insulin Isophane Suspension and 30% Regular, Human Insulin Injection), NovoLog (Insulin Aspart [rDNA origin] Inj), Nplate (romiplostim), Nutropin (Somatropin (rDNA origin) for Inj), Nutropin AQ, Nutropin Depot (Somatropin (rDNA origin) for Inj), Octreotide Acetate Injection (Sandostatin LAR), Ocrelizumab, Ofatumumab Injection (Arzerra), Olanzapine Extended Release Injectable Suspension (Zyprexa Relprevv), Omnitarg, Omnitrope (Somatropin [rDNA origin] Injection), Ondansetron Hydrochloride Injection (Zofran Injection), OptiMARK (Gadoversetamide Injection), Optiray Injection (loversol Injection), Orencia, Osmitrol Injection in Aviva (Mannitol Injection in Aviva Plastic Vessel 250), Osmitrol Injection in Viaflex (Mannitol Injection in Viaflex Plastic Vessel 250), Osteoprotegrin, Ovidrel (Choriogonadotropin Alfa Injection), Oxacillin (Oxacillin for Injection), Oxaliplatin Injection (Eloxatin), Oxytocin Injection (Pitocin), Paliperidone Palmitate Extended-Release Injectable Suspension (Invega Sustenna), Pamidronate Disodium Injection (Pam idronate Disodium Injection), Panitumumab Injection for Intravenous Use (Vectibix), Papaverine Hydrochloride Injection (Papaverine Injection), Papaverine Injection (Papaverine Hydrochloride Injection), Parathyroid Hormone, Paricalcitol Injection Fliptop Vial (Zemplar Injection), PARP Inhibitor, Pediarix, PEGIntron, Peginterferon, Pegfilgrastim, Penicillin G Benzathine and Penicillin G Procaine, Pentetate Calcium Trisodium Inj (Ca-DTPA), Pentetate Zinc Trisodium Injection (Zn-DTPA), Pepcid Injection (Famotidine Injection), Pergonal, Pertuzumab, Phentolamine Mesylate (Phentolamine Mesylate for Injection), Physostigmine Salicylate (Physostigmine Salicylate (injection)), Physostigmine Salicylate (injection) (Physostigmine Salicylate), Piperacillin and Tazobactam Injection (Zosyn), Pitocin (Oxytocin Injection), Plasma-Lyte 148 (Multiple Electrolytes Inj), Plasma-Lyte 56 and Dextrose (Multiple Electrolytes and Dextrose Injection in Viaflex, Plastic Vessel 250), PlasmaLyte, Plerixafor Injection (Mozobil), Polidocanol Injection (Asclera), Potassium Chloride, Pralatrexate Solution for Intravenous Injection (Folotyn), Pramlintide Acetate Injection (Symlin), Premarin Injection (Conjugated Estrogens for Injection), Prep kit for Technetium Tc99 Sestamibi for Injection (Cardiolite), Prevacid I.V. (Lansoprazole for Injection), Primaxin I.V. (Imipenem and Cilastatin for Injection), Prochymal, Procrit, Progesterone, ProHance (Gadoteridol Injection Solution), Prolia (Denosumab Injection), Promethazine HCl Injection (Promethazine Hydrochloride Injection), Propranolol Hydrochloride Injection (Propranolol Hydrochloride Injection), Quinidine Gluconate Injection (Quinidine Injection), Quinidine Injection (Quinidine Gluconate Injection), R-Gene 10 (Arginine Hydrochloride Injection), Ranibizumab Injection (Lucentis), Ranitidine Hydrochloride Injection (Zantac Injection), Raptiva, Reclast (Zoledronic Acid Injection), Recombivarix HB, Regadenoson Injection (Lexiscan), Reglan Injection (Metoclopramide Injection), Remicade, Renagel, Renvela (Sevelamer Carbonate), Repronex (Menotropins for Injection), Retrovir IV (Zidovudine Injection), rhApo2L/TRAIL, Ringer's and 5% Dextrose Injection (Ringers in Dextrose), Ringer's Injection (Ringers Injection), Rituxan, Rituximab, Rocephin (ceftriaxone), Rocuronium Bromide Injection (Zemuron), Roferon-A (interferon alfa-2a), Romazicon (flumazenil), Romidepsin for Injection (Istodax), Saizen (Somatropin Injection), Sandostatin LAR (Octreotide Acetate Injection), Sclerostin Ab, Sensipar (cinacalcet), Sensorcaine (Bupivacaine HCl Injections), Septocaine (Articane HCl and Epinephrine Injection), Serostim LQ (Somatropin (rDNA origin) Injection), Simponi Injection (Golimumab Injection), Sodium Acetate (Sodium Acetate Injection), Sodium Bicarbonate (Sodium Bicarbonate 5% Injection), Sodium Lactate (Sodium Lactate Injection in AVIVA), Sodium Phenylacetate and Sodium Benzoate Injection (Ammonul), Somatropin (rDNA origin) for Inj (Nutropin), Sporanox Injection (Itraconazole Injection), Stelara Injection (Ustekinumab), Stemgen, Sufenta (Sufentanil Citrate Injection), Sufentanil Citrate Injection (Sufenta), Sumavel, Sumatriptan Injection (Alsuma), Symlin, Symlin Pen, Systemic Hedgehog Antagonist, Synvisc-One (Hylan G-F 20 Single Intra-articular Injection), Tarceva, Taxotere (Docetaxel for Injection), Technetium Tc 99m, Telavancin for Injection (Vibativ), Temsirolimus Injection (Torisel), Tenormin I.V. Injection (Atenolol Inj), Teriparatide (rDNA origin) Injection (Forteo), Testosterone Cypionate, Testosterone Enanthate, Testosterone Propionate, Tev-Tropin (Somatropin, rDNA Origin, for Injection), tgAAC94, Thallous Chloride, Theophylline, Thiotepa (Thiotepa Injection), Thymoglobulin (Anti-Thymocyte Globulin (Rabbit), Thyrogen (Thyrotropin Alfa for Injection), Ticarcillin Disodium and Clavulanate Potassium Galaxy (Timentin Injection), Tigan Injection (Trimethobenzamide Hydrochloride Injectable), Timentin Injection (Ticarcillin Disodium and Clavulanate Potassium Galaxy), TNKase, Tobramycin Injection (Tobramycin Injection), Tocilizumab Injection (Actemra), Torisel (Temsirolimus Injection), Totect (Dexrazoxane for Injection, Intravenous Infusion Only), Trastuzumab-DM1, Travasol (Amino Acids (Injection)), Treanda (Bendamustine Hydrochloride Injection), Trelstar (Triptorelin Pamoate for Injectable Suspension), Triamcinolone Acetonide, Triamcinolone Diacetate, Triamcinolone Hexacetonide Injectable Suspension (Aristospan Injection 20 mg), Triesence (Triamcinolone Acetonide Injectable Suspension), Trimethobenzamide Hydrochloride Injectable (Tigan Injection), Trimetrexate Glucuronate Inj (Neutrexin), Triptorelin Pamoate for Injectable Suspension (Trelstar), Twinject, Trivaris (Triamcinolone Acetonide Injectable Suspension), Trisenox (Arsenic Trioxide Injection), Twinrix, Typhoid Vi, Ultravist (Iopromide Injection), Urofollitropin for Injection (Metrodin), Urokinase Injection (Kinlytic), Ustekinumab (Stelara Injection), Ultralente (U), Valium (diazepam), Valproate Sodium Injection (Depacon), Valtropin (Somatropin Injection), Vancomycin Hydrochloride (Vancomycin Hydrochloride Injection), Vancomycin Hydrochloride Injection (Vancomycin Hydrochloride), Vaprisol (Conivaptan Hcl Injection), VAQTA, Vasovist (Gadofosveset Trisodium Injection for Intravenous Use), Vectibix (Panitumumab Injection for Intravenous Use), Venofer (Iron Sucrose Injection), Verteporfin Inj (Visudyne), Vibativ (Telavancin for Injection), Victoza (Liraglutide [rDNA] Injection), Vimpat (lacosamide Tablet and Injection), Vinblastine Sulfate (Vinblastine Sulfate Injection), Vincasar PFS (Vincristine Sulfate Injection), Victoza, Vincristine Sulfate (Vincristine Sulfate Injection), Visudyne (Verteporfin Inj), Vitamin B-12, Vivitrol (Naltrexone XR Inj), Voluven (Hydroxyethyl Starch in Sodium Chloride Injection), Xeloda, Xenical (orlistat), Xeomin (Incobotulinumtoxin A for Injection), Xolair, Zantac Injection (Ranitidine Hydrochloride Injection), Zemplar Injection (Paricalcitol Injection Fliptop Vial), Zemuron (Rocuronium Bromide Injection), Zenapax (daclizumab), Zevalin, Zidovudine Injection (Retrovir IV), Zithromax Injection (Azithromycin), Zn-DTPA (Pentetate Zinc Trisodium Injection), Zofran Injection (Ondansetron Hydrochloride Injection), Zingo, Zoledronic Acid for Inj (Zometa), Zoledronic Acid Injection (Reclast), Zometa (Zoledronic Acid for Inj), Zosyn (Piperacillin and Tazobactam Injection), Zyprexa Relprevv (Olanzapine Extended Release Injectable Suspension) and combinations thereof.

## Claims

1. A method for manufacturing an injector device (100), the method comprising:
positioning a stopper (40) of the injector device (100) in a tubular member (20, 4266) such that an outer side (244) of the stopper (40) is engaged with an inner surface (124) of the tubular member (20, 4266); and
modifying the outer side of a stopper (40) by causing relative motion between the stopper (40) and the tubular member (20, 4266), the relative motion including one or both of rotational motion and vibrational motion; wherein the relative motion causes material from the outer side (244) of the stopper (40) to be transferred to the inner surface (124) of the tubular member (20, 4266).

2. The method of claim 1, wherein the tubular member (20, 4266) is a barrel (20) of the injector device (100), or
wherein the tubular member (20, 4266)) is a vent tube (4264) configured to be inserted into a barrel (20) of the injector device, the vent tube (4264) being configured for delivery of the stopper (40) into a barrel (20) of the injector device (100).

3. The method of any preceding claim, wherein at least one of:
the outer side (244) of the stopper (40) defines a rib (300) and the relative motion between the stopper (40) and the tubular member (20, 4266) results in localized heating at the rib (300);
the outer side (244) of the stopper includes wrinkles (362) and the relative motion causes a reduction in the wrinkles (362).

4. The method of any preceding claim, wherein at least one of:
the relative motion causes a reduction in roughness of the outer side (244) of the stopper (40);
the relative motion between the stopper (40) and the tubular member (20, 4266) includes a longitudinal component.

5. The method of any preceding claim, wherein the outer side (244) of the stopper (40) defines a roughness in a longitudinal direction and a circumferential direction, and further wherein the relative motion causes a reduction in roughness in the longitudinal direction and/or the circumferential direction.

6. The method of any preceding claim, wherein at least one of:
the outer side (244) of the stopper (40) includes polymeric material and relative motion induces polymeric movement of the polymeric material; and
wherein the outer side (244) of the stopper (40) includes a fluoropolymer material.

7. The method of any preceding claim, wherein the stopper (40) includes a barrier (242) formed of a first material and a body (240) formed of a second material, the barrier (242) being coupled to the body (240), wherein the barrier (242) includes a fluoropolymer material.

8. A method for manufacturing an injector device (100), the method comprising:
positioning a stopper (40) of the injector device in a vent tube (4264);
inserting the vent tube (4264) into a barrel (20) of the injector device;
delivering the stopper (40) from the vent tube (4264) into the barrel (20) of the injector device such that an outer side (244) of the stopper (40) is engaged with an inner surface (124) of the barrel (20) to define a seal interface (702) between the outer side (244) of the stopper (40) and the inner surface (124) of the barrel (20); and
inducing relative motion between the stopper (40) and the barrel (20) to enhance the seal interface (702) between the outer side (244) of the stopper (40) and the inner surface (124) of the barrel (20);
wherein enhancing the seal interface (702) between the outer side (244) of the stopper (40) and the inner surface (124) of the barrel (20) includes transferring material from the outer side (244) of the stopper (40) to the inner side (124) of the barrel (20).

9. The method of claim 8, wherein at least one of:
the relative motion between the stopper (40) and the tubular member (4266) includes a rotational component;
the relative motion between the stopper (40) and the tubular member(4266) includes a longitudinal component.

10. The method of any of claims 8 or 9, wherein at least one of:
the outer side (244) of the stopper (40) includes a polymeric material and enhancing the seal interface between the outer side (244) of the stopper (40) and the inner surface (124) of the barrel (20) includes inducing polymeric movement of the polymeric material at the seal interface; and
the outer side (244) of the stopper (40) defines a rib (300) and the seal interface includes the rib (300) of the stopper (40).

11. The method of any of claims 8 to 10, wherein after the stopper (40) is delivered from the vent tube (4264) into the barrel (20) of the injector device (100), the outer side (244) of the stopper (40) includes wrinkling (362) at the seal interface (702), and further wherein enhancing the seal interface between the outer side (244) of the stopper (40) and the inner surface (124) of the barrel (20) includes reducing wrinkling at the seal interface (702), and/or
wherein the stopper (40) includes a body (240) and a barrier (242) coupled to the body (240), the barrier (242) being formed of a fluoropolymer material, and further wherein enhancing the seal interface (702) between the outer side (244) of the stopper (40) and the inner surface (124) of the barrel (20) includes causing localized heating at the seal interface (702).

12. An injector device (100) comprising:
a stopper (40) having an outer side (244) and including a body (240) and a barrier (242) formed of a material different than that of the body (240), the barrier (240) being coupled to the body (242), the barrier (242) defining at least a portion of the outer side (244) of the stopper (40), and wherein the barrier (242) includes a fluoropolymer material; and
a barrel (20) having an inner surface (124) engaged with outer side (244) of the stopper (40) to define a seal interface (702), the inner surface (124) of the barrel (20) including a deposited material at the seal interface (702) corresponding to the material of the barrier (242) such that the seal interface (702) is defined by the deposited material and the material of the barrier (242), the deposited material having a directional orientation; wherein the deposited material at the seal interface is material that has been transferred from the outer side (244) of the stopper (40) to the inner surface of the barrel (20) by relative motion between the stopper (40) and the barrel (20).

13. The injector device of claim 12, wherein at least one of:
the directional orientation includes a circumferential component;
the directional orientation of the deposited material is defined by rows of PTFE chains aligned in a common direction.

14. The injector device of any of claims 12 or 13, wherein the outer side (244) of the stopper (40) includes at least one of a micro rib (400A) and a macro rib (300) at the seal interface (702).

15. The injector device of any of claims 12 to 14, wherein at least one of:
the deposited material fills one or more defects in the inner surface (124) of the barrel (20);
the deposited material is only located at the seal interface (702);
the deposited material defines one or more circumferential bands on the inside of the barrel (20).

16. The injector device of any of claims 12 to 15, wherein the seal interface (702) corresponds to one or more circumferential bands where the outer side (244) of the stopper (40) engages the inner surface (124) of the barrel (20).

## Patentansprüche

1. Verfahren zur Herstellung einer Injektorvorrichtung (100), wobei das Verfahren Folgendes umfasst:
Positionieren eines Stopfens (40) der Injektorvorrichtung (100) in einem rohrförmigen Element (20, 4266) derart, dass eine Außenseite (244) des Stopfens (40) mit einer Innenfläche (124) des rohrförmigen Elements (20, 4266) in Eingriff steht; und
Modifizieren der Außenseite eines Stopfens (40) durch Bewirken einer Relativbewegung zwischen dem Stopfen (40) und dem rohrförmigen Element (20, 4266), wobei die Relativbewegung eines oder beide von Drehbewegung und Vibrationsbewegung beinhaltet; wobei die Relativbewegung bewirkt, dass Material von der Außenseite (244) des Stopfens (40) auf die Innenfläche (124) des rohrförmigen Elements (20, 4266) übertragen wird.

2. Verfahren nach Anspruch 1, wobei das rohrförmige Element (20, 4266) ein Zylinder (20) der Injektorvorrichtung (100) ist, oder
wobei das rohrförmige Element (20, 4266) ein Entlüftungsrohr (4264) ist, das dazu konfiguriert ist, in einen Zylinder (20) der Injektorvorrichtung eingeführt zu werden, wobei das Entlüftungsrohr (4264) zur Zuführung des Stopfens (40) in einen Zylinder (20) der Injektorvorrichtung (100) konfiguriert ist.

3. Verfahren nach einem vorhergehenden Anspruch, wobei zumindest eines von Folgendem gilt:
die Außenseite (244) des Stopfens (40) definiert eine Rippe (300) und die Relativbewegung zwischen dem Stopfen (40) und dem rohrförmigen Element (20, 4266) führt zu einer lokalisierten Erwärmung an der Rippe (300);
die Außenseite (244) des Stopfens beinhaltet Falten (362) und die Relativbewegung bewirkt eine Verringerung der Falten (362).

4. Verfahren nach einem vorhergehenden Anspruch, wobei zumindest eines von Folgendem gilt:
die Relativbewegung bewirkt eine Verringerung der Rauheit der Außenseite (244) des Stopfens (40);
die Relativbewegung zwischen dem Stopfen (40) und dem rohrförmigen Element (20, 4266) beinhaltet eine Längskomponente.

5. Verfahren nach einem vorhergehenden Anspruch, wobei die Außenseite (244) des Stopfens (40) eine Rauheit in einer Längsrichtung und einer Umfangsrichtung definiert und ferner wobei die Relativbewegung eine Verringerung der Rauheit in der Längsrichtung und/oder der Umfangsrichtung bewirkt.

6. Verfahren nach einem vorhergehenden Anspruch, wobei zumindest eines von Folgendem gilt:
die Außenseite (244) des Stopfens (40) beinhaltet Polymermaterial und eine Relativbewegung induziert eine Polymerbewegung des Polymermaterials; und
wobei die Außenseite (244) des Stopfens (40) ein Fluorpolymermaterial beinhaltet.

7. Verfahren nach einem vorhergehenden Anspruch, wobei der Stopfen (40) eine Barriere (242), die aus einem ersten Material gebildet ist, und einen Körper (240), der aus einem zweiten Material gebildet ist, beinhaltet, wobei die Barriere (242) an den Körper (240) gekoppelt ist, wobei die Barriere (242) ein Fluorpolymermaterial beinhaltet.

8. Verfahren zur Herstellung einer Injektorvorrichtung (100), wobei das Verfahren Folgendes umfasst:
Positionieren eines Stopfens (40) der Injektorvorrichtung in einem Entlüftungsrohr (4264);
Einführen des Entlüftungsrohrs (4264) in einen Zylinder (20) der Injektorvorrichtung;
Zuführen des Stopfens (40) von dem Entlüftungsrohr (4264) in den Zylinder (20) der Injektorvorrichtung, derart, dass eine Außenseite (244) des Stopfens (40) mit einer Innenfläche (124) des Zylinders (20) in Eingriff steht, um eine Dichtungsschnittstelle (702) zwischen der Außenseite (244) des Stopfens (40) und der Innenfläche (124) des Zylinders (20) zu definieren; und
Induzieren einer Relativbewegung zwischen dem Stopfen (40) und dem Zylinder (20), um die Dichtungsschnittstelle (702) zwischen der Außenseite (244) des Stopfens (40) und der Innenfläche (124) des Zylinders (20) zu verbessern;
wobei das Verbessern der Dichtungsschnittstelle (702) zwischen der Außenseite (244) des Stopfens (40) und der Innenfläche (124) des Zylinders (20) das Übertragen von Material von der Außenseite (244) des Stopfens (40) auf die Innenseite (124) des Zylinders (20) beinhaltet.

9. Verfahren nach Anspruch 8, wobei zumindest eines von Folgendem gilt:
die Relativbewegung zwischen dem Stopfen (40) und dem rohrförmigen Element (4266) beinhaltet eine Drehkomponente;
die Relativbewegung zwischen dem Stopfen (40) und dem rohrförmigen Element (4266) beinhaltet eine Längskomponente.

10. Verfahren nach einem der Ansprüche 8 oder 9, wobei zumindest eines von Folgendem gilt:
die Außenseite (244) des Stopfens (40) beinhaltet ein Polymermaterial und das Verbessern der Dichtungsschnittstelle zwischen der Außenseite (244) des Stopfens (40) und der Innenfläche (124) des Zylinders (20) beinhaltet das Induzieren einer Polymerbewegung des Polymermaterials an der Dichtungsschnittstelle; und
die Außenseite (244) des Stopfens (40) definiert eine Rippe (300) und die Dichtungsschnittstelle beinhaltet die Rippe (300) des Stopfens (40).

11. Verfahren nach einem der Ansprüche 8 bis 10, wobei nach dem Zuführen des Stopfens (40) von dem Entlüftungsrohr (4264) in den Zylinder (20) der Injektorvorrichtung (100) die Außenseite (244) des Stopfens (40) eine Faltenbildung (362) an der Dichtungsschnittstelle (702) beinhaltet, und ferner wobei das Verbessern der Dichtungsschnittstelle zwischen der Außenseite (244) des Stopfens (40) und der Innenfläche (124) des Zylinders (20) eine Verringerung der Faltenbildung an der Dichtungsschnittstelle (702) beinhaltet, und/oder
wobei der Stopfen (40) einen Körper (240) und eine Barriere (242) beinhaltet, die an den Körper (240) gekoppelt ist, wobei die Barriere (242) aus einem Fluorpolymermaterial gebildet ist, und ferner wobei das Verbessern der Dichtungsschnittstelle (702) zwischen der Außenseite (244) des Stopfens (40) und der Innenfläche (124) des Zylinders (20) das Bewirken einer lokalisierten Erwärmung an der Dichtungsschnittstelle (702) beinhaltet.

12. Injektorvorrichtung (100), umfassend:
einen Stopfen (40), der eine Außenseite (244) aufweist und einen Körper (240) und eine Barriere (242) beinhaltet, die aus einem Material gebildet ist, das sich von dem des Körpers (240) unterscheidet, wobei die Barriere (240) an den Körper (242) gekoppelt ist, wobei die Barriere (242) zumindest einen Abschnitt der Außenseite (244) des Stopfens (40) definiert, und wobei die Barriere (242) ein Fluorpolymermaterial beinhaltet; und
einen Zylinder (20), der eine Innenfläche (124) aufweist, die mit der Außenseite (244) des Stopfens (40) in Eingriff steht, um eine Dichtungsschnittstelle (702) zu definieren, wobei die Innenfläche (124) des Zylinders (20) ein abgeschiedenes Material an der Dichtungsschnittstelle (702) beinhaltet, das dem Material der Barriere (242) entspricht, derart, dass die Dichtungsschnittstelle (702) durch das abgeschiedene Material und das Material der Barriere (242) definiert ist, wobei das abgeschiedene Material eine Richtungsorientierung aufweist; wobei das abgeschiedene Material an der Dichtungsschnittstelle Material ist, das von der Außenseite (244) des Stopfens (40) auf die Innenfläche des Zylinders (20) durch eine Relativbewegung zwischen dem Stopfen (40) und dem Zylinder (20) übertragen worden ist.

13. Injektorvorrichtung nach Anspruch 12, wobei zumindest eines von Folgendem gilt:
die Richtungsorientierung beinhaltet eine Umfangskomponente;
die Richtungsorientierung des abgeschiedenen Materials ist durch Reihen von PTFE-Ketten definiert, die in einer gemeinsamen Richtung ausgerichtet sind.

14. Injektorvorrichtung nach einem der Ansprüche 12 oder 13, wobei die Außenseite (244) des Stopfens (40) zumindest eine von einer Mikrorippe (400A) und einer Makrorippe (300) an der Dichtungsschnittstelle (702) beinhaltet.

15. Injektorvorrichtung nach einem der Ansprüche 12 bis 14, wobei zumindest eines von Folgendem gilt:
das abgeschiedene Material füllt einen oder mehrere Defekte in der Innenfläche (124) des Zylinders (20) aus;
das abgeschiedene Material befindet sich nur an der Dichtungsschnittstelle (702);
das abgeschiedene Material definiert ein oder mehrere Umfangsbänder an der Innenseite des Zylinders (20).

16. Injektorvorrichtung nach einem der Ansprüche 12 bis 15, wobei die Dichtungsschnittstelle (702) einem oder mehreren Umfangsbändern entspricht, wobei die Außenseite (244) des Stopfens (40) mit der Innenfläche (124) des Zylinders (20) in Eingriff steht.

## Revendications

1. Procédé permettant la fabrication d'un dispositif d'injecteur (100), le procédé comprenant :
le positionnement d'un bouchon (40) du dispositif d'injecteur (100) dans un élément tubulaire (20, 4266)
de sorte qu'un côté externe (244) du bouchon (40) soit en prise avec une surface interne (124) de l'élément tubulaire (20, 4266) ; et
la modification du côté externe d'un bouchon (40) en entraînant un mouvement relatif entre le bouchon (40) et l'élément tubulaire (20, 4266), ledit mouvement relatif comprenant un mouvement de rotation et/ou un mouvement vibratoire ; ledit mouvement relatif amenant le matériau provenant du côté externe (244) du bouchon (40) à être transféré vers la surface interne (124) de l'élément tubulaire (20, 4266).

2. Procédé de la revendication 1, ledit élément tubulaire (20, 4266) étant un cylindre (20) du dispositif d'injecteur (100), ou
ledit élément tubulaire (20, 4266) étant un tube d'évent (4264) conçu pour être inséré dans un cylindre (20) du dispositif d'injecteur, le tube d'évent (4264) étant conçu pour la distribution du bouchon (40) dans un cylindre (20) du dispositif d'injecteur (100).

3. Procédé d'une quelconque revendication précédente, au moins un parmi :
ledit côté externe (244) du bouchon (40) définissant une nervure (300) et ledit mouvement relatif entre le bouchon (40) et l'élément tubulaire (20, 4266) engendrant un chauffage localisé au niveau de la nervure (300) ;
ledit côté externe (244) du bouchon comprenant des plis (362) et ledit mouvement relatif entraînant une réduction des plis (362).

4. Procédé d'une quelconque revendication précédente, au moins un parmi :
ledit mouvement relatif entraînant une réduction de la rugosité du côté externe (244) du bouchon (40) ;
ledit mouvement relatif entre le bouchon (40) et l'élément tubulaire (20, 4266) comprenant un composant longitudinal.

5. Procédé d'une quelconque revendication précédente, ledit côté externe (244) du bouchon (40) définissant une rugosité dans une direction longitudinale et une direction circonférentielle, et en outre ledit mouvement relatif entraînant une réduction de la rugosité dans la direction longitudinale et/ou la direction circonférentielle.

6. Procédé d'une quelconque revendication précédente, au moins un parmi :
ledit côté externe (244) du bouchon (40) comprenant un matériau polymère et un mouvement relatif engendrant un mouvement polymère du matériau polymère ; et
ledit côté externe (244) du bouchon (40) comprenant un matériau en polymère fluoré.

7. Procédé d'une quelconque revendication précédente, ledit bouchon (40) comprenant une barrière (242) formée d'un premier matériau et un corps (240) formé d'un second matériau, la barrière (242) étant couplée au corps (240), ladite barrière (242) comprenant un matériau en polymère fluoré.

8. Procédé permettant la fabrication d'un dispositif d'injecteur (100), le procédé comprenant :
le positionnement d'un bouchon (40) du dispositif d'injecteur dans un tube d'évent (4264) ;
l'insertion du tube d'évent (4264) dans un cylindre (20) du dispositif d'injecteur ;
la distribution du bouchon (40) à partir du tube d'évent (4264) dans le cylindre (20) du dispositif d'injecteur de sorte qu'un côté externe (244) du bouchon (40) soit en prise avec une surface interne (124) du cylindre (20) pour définir une interface d'étanchéité (702) entre le côté externe (244) du bouchon (40) et la surface interne (124) du cylindre (20) ; et
l'engendrement d'un mouvement relatif entre le bouchon (40) et le cylindre (20) pour améliorer l'interface d'étanchéité (702) entre le côté externe (244) du bouchon (40) et la surface interne (124) du cylindre (20) ;
ladite amélioration de l'interface d'étanchéité (702) entre le côté externe (244) du bouchon (40) et
la surface interne (124) du cylindre (20) comprenant le transfert de matériau du côté externe (244) du bouchon (40) vers le côté interne (124) du cylindre (20).

9. Procédé de la revendication 8, au moins un parmi :
ledit mouvement relatif entre le bouchon (40) et l'élément tubulaire (4266) comprenant un composant rotatif ;
ledit mouvement relatif entre le bouchon (40) et l'élément tubulaire (4266) comprenant un composant longitudinal.

10. Procédé de l'une quelconque des revendications 8 ou 9, au moins un parmi :
ledit côté externe (244) du bouchon (40) comprenant un matériau polymère et ladite amélioration de l'interface d'étanchéité entre le côté externe (244) du bouchon (40) et la surface interne (124) du cylindre (20) comprenant l'engendrement d'un mouvement polymère du matériau polymère au niveau de l'interface d'étanchéité ; et
ledit côté externe (244) du bouchon (40) définissant une nervure (300) et ladite interface d'étanchéité comprenant la nervure (300) du bouchon (40).

11. Procédé de l'une quelconque des revendications 8 à 10, après que le bouchon (40) a été distribué à partir du tube d'évent (4264) dans le cylindre (20) du dispositif d'injecteur (100), ledit côté externe (244) du bouchon (40) comprenant des plis (362) au niveau de l'interface d'étanchéité (702), et en outre ladite amélioration de l'interface d'étanchéité entre le côté externe (244) du bouchon (40) et la surface interne (124) du cylindre (20) comprenant la réduction des plis au niveau de l'interface d'étanchéité (702), et/ou
ledit bouchon (40) comprenant un corps (240) et une barrière (242) couplée au corps (240), la barrière (242) étant formée d'un matériau en polymère fluoré, et en outre ladite amélioration de l'interface d'étanchéité (702) entre le côté externe (244) du bouchon (40) et la surface interne (124) du cylindre (20) comprenant l'entraînement d'un chauffage localisé au niveau de l'interface d'étanchéité (702).

12. Dispositif d'injecteur (100), comprenant :
un bouchon (40) possédant un côté externe (244) et comprenant un corps (240) et une barrière (242) formée d'un matériau différent de celui du corps (240), la barrière (240) étant couplée au corps (242), la barrière (242) définissant au moins une partie du côté externe (244) du bouchon (40), et ladite barrière (242) comprenant un matériau en polymère fluoré ; et
un cylindre (20) possédant une surface interne (124) en prise avec le côté externe (244) du bouchon (40) pour définir une interface d'étanchéité (702), la surface interne (124) du cylindre (20) comprenant un matériau déposé au niveau de l'interface d'étanchéité (702) correspondant au matériau de la barrière (242) de sorte que l'interface d'étanchéité (702) soit définie par le matériau déposé et le matériau de la barrière (242), le matériau déposé possédant une orientation directionnelle ; ledit matériau déposé au niveau de l'interface d'étanchéité étant un matériau qui a été transféré du côté externe (244) du bouchon (40) à la surface interne du cylindre (20) par un mouvement relatif entre le bouchon (40) et le cylindre (20).

13. Dispositif d'injecteur de la revendication 12, au moins un parmi :
ladite orientation directionnelle comprenant une composante circonférentielle ;
ladite orientation directionnelle du matériau déposé étant définie par des rangées de chaînes PTFE alignées dans une direction commune.

14. Dispositif d'injecteur selon l'une quelconque des revendications 12 ou 13, ledit côté externe (244) du bouchon (40) comprenant au moins une micro nervure (400A) et une macro nervure (300) au niveau de l'interface d'étanchéité (702).

15. Dispositif d'injecteur de l'une quelconque des revendications 12 à 14, au moins un parmi :
ledit matériau déposé remplissant un ou plusieurs défauts dans la surface interne (124) du cylindre (20) ;
ledit matériau déposé étant uniquement situé au niveau de l'interface d'étanchéité (702) ;
ledit matériau déposé définissant une ou plusieurs bandes circonférentielles à l'intérieur du cylindre (20).

16. Dispositif d'injecteur de l'une quelconque des revendications 12 à 15, ladite interface d'étanchéité (702) correspondant à une ou plusieurs bandes circonférentielles où le côté externe (244) du bouchon (40) vient en prise avec la surface interne (124) du cylindre (20).
